(19) Europäisches Patentamt / European Patent Office / Office européen des brevets

(11) EP 4 436 666 B1

(12) # EUROPEAN PATENT SPECIFICATION

(45) Date of publication and mention of the grant of the patent:
**13.08.2025 Bulletin 2025/33**

(21) Application number: **22814188.3**

(22) Date of filing: **23.11.2022**

(51) International Patent Classification (IPC):
***A61P 31/00*** (2006.01) ***C01B 32/05*** (2017.01)
***A61P 31/20*** (2006.01) ***A61P 31/14*** (2006.01)
***A61P 31/12*** (2006.01) ***A61P 31/04*** (2006.01)
***A61K 33/44*** (2006.01) ***C01B 32/15*** (2017.01)

(52) Cooperative Patent Classification (CPC):
**A61P 31/00; A61P 31/04; A61P 31/12; A61P 31/14; A61P 31/20; C01B 32/15**

(86) International application number:
**PCT/IB2022/061324**

(87) International publication number:
**WO 2023/095013 (01.06.2023 Gazette 2023/22)**

(54) **NEW ANTI MICROBIAL CARBON DOTS**

NEUE ANTI-MIKROBISCHE KOHLENSTOFFPUNKTE

NOUVEAUX POINTS DE CARBONE ANTIMICROBIENS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **23.11.2021 IT 202100029591**

(43) Date of publication of application:
**02.10.2024 Bulletin 2024/40**

(73) Proprietors:
- **FONDAZIONE CENTRO SERVIZI ALLA PERSONA**
  **09020 Villamar SU (IT)**
- **Ecoaida S.r.l.**
  **07041 Alghero SS (IT)**
- **Malfatti, Luca**
  **07041 Alghero SS (IT)**
- **Innocenzi, Plinio**
  **07041 Alghero (SS) (IT)**

(72) Inventors:
- **DE FORNI, Davide**
  **07041 Alghero SS (IT)**
- **PODDIGHE, Matteo**
  **07100 Sassari SS (IT)**
- **STAGI, Luigi**
  **09020 Siddi SS (IT)**
- **INNOCENZI, Plinio**
  **07041 Alghero SS (IT)**
- **MALFATTI, Luca**
  **07041 Alghero SS (IT)**

(74) Representative: **Predazzi, Valentina**
**Società Italiana Brevetti S.p.A.**
**Piazza di Pietra, 39**
**00186 Roma (IT)**

(56) References cited:
- **JAHAN SHANAZ ET AL: "Oxidative Synthesis of Highly Fluorescent Boron/Nitrogen Co-Doped Carbon Nanodots Enabling Detection of Photosensitizer and Carcinogenic Dye", ANALYTICAL CHEMISTRY, vol. 85, no. 21, 5 November 2013 (2013-11-05), US, pages 10232 - 10239, XP055934787, ISSN: 0003-2700, DOI: 10.1021/ac401949k**
- **DAS SMITA ET AL: "Carbon Dots: An Emerging Smart Material for Analytical Applications", MICROMACHINES, vol. 12, no. 1, 15 January 2021 (2021-01-15), pages 84, XP055865291, DOI: 10.3390/mi12010084**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 436 666 B1

- **KOVÁCOVÁ MÁRIA ET AL: "Carbon Quantum Dots Modified Polyurethane Nanocomposite as Effective Photocatalytic and Antibacterial Agents", vol. 4, no. 12, 27 September 2018 (2018-09-27), pages 3983 - 3993, XP055934718, ISSN: 2373-9878, Retrieved from the Internet <URL:https://pubs.acs.org/doi/pdf/10.1021/acsbiomaterials.8b00582> DOI: 10.1021/acsbiomaterials.8b00582**
- **NIE XIAOLIN ET AL: "Carbon quantum dots: A bright future as photosensitizers for in vitro antibacterial photodynamic inactivation", JOURNAL OF PHOTOCHEMISTRY AND PHOTOBIOLOGY B: BIOLOGY, ELSEVIER SCIENCE S.A., BASEL, CH, vol. 206, 23 March 2020 (2020-03-23), XP086124270, ISSN: 1011-1344, [retrieved on 20200323], DOI: 10.1016/J.JPHOTOBIOL.2020.111864**
- **INNOCENZI PLINIO ET AL: "Carbon-based antiviral nanomaterials: graphene, C-dots, and fullerenes. A perspective", vol. 11, no. 26, 8 July 2020 (2020-07-08), United Kingdom, pages 6606 - 6622, XP055878064, ISSN: 2041-6520, Retrieved from the Internet <URL:https://pubs.rsc.org/en/content/articlepdf/2020/sc/d0sc02658a> DOI: 10.1039/D0SC02658A**

## Description

The present invention relates to new antiviral carbon dots, compositions comprising said carbon dots, materials and devices coated with said carbon dots, said nanoparticles for medical use, methods for the preparation of said carbon dots, compositions, materials and devices.

STATE OF THE ART

Infectious diseases account for 20% of global mortality and viruses are responsible for about one third of these deaths. Among the most common causes of death are lower respiratory tract infections and human immunodeficiency viruses (HIV) and represent a major expense for national health systems. In addition to these well-known viruses, new ones emerge every year (e.g., the more recent SARS-CoV-2). The best approach to fight viral infections is, in the first instance preventing the spread of infections by respecting a number of hygienic and behavioural rules, and in terms of therapy represented by vaccines, but there is a limited number of effective vaccines and they are often not available in all parts of the world.

A further key role in a successful response in this fight is represented by antiviral drugs and/or compounds. However, said drugs are often limited by the virus specificity, and are in many cases ineffective with the emergence of new and unknown viral strains.

Prevention of spread of diseases, such as e.g. SARS-CoV-2; is enhanced by a number of actions. Wearing suitable personal protection equipment and keeping surfaces and environments clean and disinfected is a relevant part in the fight to infectious diseases spread. In the case of a pandemics, the need of strongly active antiviral compounds allowing fast and effective inactivation of the virus in the air as well as on surfaces (animated and non-animated) has been strongly felt.

Prevention, including but not limited to prevention through vaccination, has proven to be the only effective weapon against spread of infectious diseases, this being particularly true for diseases for which no effective treatments are available or globally available, and for diseases living strong damages in the body notwithstanding the effectiveness of treatments for fighting the infectious agent.

Effective and non-toxic antimicrobial (in particular virucidal, antifungal and/or bactericidal) compounds are a constant need, in particular, compounds that act through mechanisms that are effective on classes of pathogenic agents rather than pathogen specific are preferred in view of the constant evolution of new pathogens and of their potential devastating effects as the SARS-CoV-2 global spread has demonstrated.

In recent years, the lively research on carbon-based nanomaterials has revealed that some systems have properties of interest for nanomedical purposes. These include carbon dots (C-dots).

Carbon dots (CDs) are a large family of carbon-based materials including amorphous carbon nanoparticles, partially graphitized core-shell carbon nanoparticles, amorphous fluorescent polymeric nanoparticles, and graphene quantum dots. The CDs synthesis is achieved either via top-down methods, trough laser ablation of graphitic targets or electrochemical oxidation of graphite electrodes, either via bottom-up methods, by carbonization of organic precursors. The latter pathway, in particular, enables an easy CD tailoring through an appropriate choice of chemical reagents. In the previous years a large attention has been devoted to the application of CDS as antibacterial agents. The antibacterial effect has been attributed so far to the functional groups on the surface, which in turn are responsible for their extraordinary optical properties as fluorophores. CDs, in fact, have shown an active role against infections which appeared further increased when they are exposed to UV light sources (Innocenzi Chem Sci 2020). This evidence has pushed the scientific community to hypothesize the production of reactive oxygen species ROS, when CDs are photoexcited. Since that hypothesis, most part of the CDs have been therefore recognized as efficient and biocompatible photosensitizer nanomaterials, capable of producing reactive oxygen species under excitation by UV or, in a few cases, visible lights. In particular, CDs with high phosphorescence yield have proven to be efficient Singlet oxygen (SO) photosensitizer, showing promising characteristics as UV enhanced antimicrobials (ACS Biomater. Sci. Eng. 2018, 4, 12, 3983-3993). Among the different ROS, in fact, SO represents a powerful antibacterial and antimicrobial agent (Muller-Breitkreutz J Photochem PhotoBiol B 1995, Ogilby Chem Soc Rev 2010) that causes an alteration in the organization of the membranes. SO is also an effective broad-spectrum antiviral agent that directly acts on the envelope.

Electrochemically produced graphene quantum dots (GQD) have been already proved to generate ROS when photoexcited in the visible light (470 nm, 1 W). The photogenerated ROS have been in turn capable of killing methicillin-resistant Staphylococcus aureus and Escherichia coli. In contrast with the response of graphene or graphene oxide, which can kill tumor cells by releasing heat, the irradiated GQD do not cause a significant temperature increase in the bacterial suspensions (B.Z. Ristic et al. / Biomaterials 35 (2014) 4428- 4435) while produce a significative increase in ROS concentration. Most part of the research published so far attributes the pro-oxidant activity of CDs to the SO formation. For the GQDs, in particular, it has been proposed that SO is generated through photoexcitation via energy transfer and electron transfer pathways. This implies that both hydroxyl radicals and superoxide anions arise from creation of electron-hole pairs and charge separation, respectively reacting with surrounding molecular oxygen or H2O (ACS Nano 2016, 10, 9, 8690-8699). At the moment, there is not a comprehensive explanation of the ROS production induced by CDs' photoexcitation, however some results indicate that the ROS generation of can be related to oxygen functional groups in the CDS structure. The removal of oxygen functional groups, especially for ketonic carbonyl groups on the surface of GQDs, has been proved to inhibit the ROS formation of GQDs in cells and lower the photoinduced cytotoxicity. (Chem. Commun., 2017,53, 10588-10591).

Shanaz et al 2013 (Shanaz et al "Oxidative Synthesis of Highly Fluorescent Boron/Nitrogen Co-Doped Carbon Nanodots Enabling Detection of Photosensitizer and Carcinogenic Dye" Analytical Chemistry 85(21) 2013) discloses the synthesis of Carbon dots starting from boric acid and N-(4-hydroxyphenyl)glycine under hydrothermal conditions. The document discloses various characterisation parameters of said Carbon Dots.

Das et al 2021 (Das et al "Carbon Dots: An Emerging Smart Material for Analytical Application" Micromachines 2021 2(1):84) reports on a number of different Carbon dots synthesis obtained form hydrothermal, solvothermal, microwave and pure thermal synthesis, inducing degradation reaction such as thermolysis or combustion. Among the different protocols, a method of synthesis involving only glycine and citric acid to form Carbon dots is disclosed.

Kováčová et al 2018 (Kováčová et al "Carbon Quantum Dots Modified Polyurethane Nanocomposite as Effective Photocatalytic and Antibacterial Agents" ACS Biomater. Sci. Eng. 2018, 4, 12, 3983-3993) discloses the synthesis of Carbon dots-based nanocomposite obtained by embedding the nanoparticles in polyurethane polymer matrices. The Carbon dots are obtained by hydrothermal synthesis of a tri-block copolymer in presence of phosphoric acid.

Xiaolin Nie et al 2020 (Xiaolin Nie et al "Carbon quantum dots: A bright future as photosensitizers for in vitro antibacterial photodynamic inactivation" Photochem Photobiol B. 2020 Mar 23;206:111864) reports a modified version of an already published Carbon dots synthesis. The protocol involves a one-pot solvothermal procedure employing a mass ratio of citric acid to 1,5-diaminonaphthalene of 1:2. The document focues on carbon dots antibacterial activities

Innocenzi and Stagi 2020 (Innocenzi and Stagi "Carbon-based antiviral nanomaterials: graphene, C-dots, and fullerenes. A perspective" Chem Sci. 2020 Jul 14; 11 (26): 6606-6622) is a review on Carbon-based antiviral nanomaterials such as Carbon dots, graphene, fullerenes and nanodiamons as alternative innovative antiviral systems.

C-dots have been extensively investigated for their excellent properties in photonics, optoelectronics and photocatalysis. In addition, C-dots appear to play a decisive role both as luminescent markers and as active systems against viral agents. One of the key features of C-dots is the ease of implementation and low cost, which allows the realisation of systems controlled in chemical composition and size, typically below 10 nm. In a common synthesis, a carbon-based precursor undergoes controlled thermal decomposition under wet or dry conditions. The product can be engineered by reacting nitrogen sources with carbon precursors or by performing post-synthesis functionalisation. Studies performed on Vero cells, MOLT-4 A549, MARC-145, PK-15 and many others, confirm the excellent biocompatibility of C-dots, being non-cytotoxic even at high concentrations of several hundred $\mu g\ mL^{-1}$. C-dots with controlled morphology, derived from various monomers, have been shown to be effective against HSV-1, HIV-1, PRRSV, PRV, HCoV-229E and flaviviruses (JEV). Although there are a number of encouraging results, the mechanism of virus inhibition by C-dots is controversial and still debated. In particular, it is believed that C-dots with specific functional groups on the surface may interact with the cell membrane, preventing virions from attacking the system. More recently, on the other hand, benzoxazine-derived C-dots have been shown to be effective in hindering viral activity by interacting directly with virions, constituting the first true dots system capable of actively opposing viral particles and displaying broad-spectrum antiviral activity.

Based on the state of the art, it is hence evident that CDs can have extremely variable antimicrobial effects depending on the exact CD type and that the effectiveness of the CD as antimicrobic (e.g. antibacterial and/or antiviral and/or antifungal) effect is not predictable a priori.

Given the increasing urge to enhance infectious diseases spread in the more environmental and safety friendly way possible developing new compounds fulfilling said requirement and that are not likely to elicit resistance in the pathogens is of extreme importance for the global health, including both plants and animal kingdoms.

## SUMMARY OF THE INVENTION

The authors of the present invention explored the potential of 3 different types of CDs as antiviral materials against SARS-COV 2 obtained by carbonization of similar precursors under hydrothermal conditions. Among the multiple CDs precursor which have been used so far in the literature, the inventors have selected citric acid, probably the most used precursors for the bottom-up synthesis of CDs, 1,5-diaminonaphthalene, and glycine, an amino acid which can be thermally polymerized at low temperature (Scheme 1).

The glycine according to the present invention is a non-functionalised glycine.

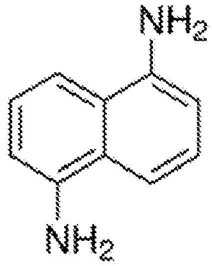

1,5-diaminonaphthalene

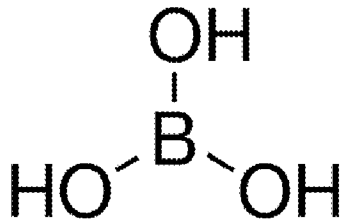

boric acid

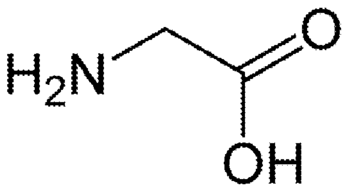

glycine

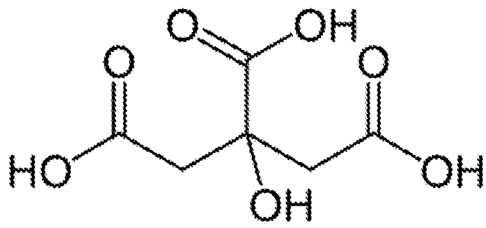

citric acid

Scheme 1.

The authors have also compared two CDs obtained from the same precursors using two different acids (hydrochloric or boric acid) with CDs obtained substituting one of the two precursors in the preparation process and have surprisingly found that the use of a specific combination of precursors together with one or the other of said selected acidsprovided CDs with a powerful virucidal effect upon irradiation with either UV or visible light.

In particular, even depending on the acid selected CDs prepared with boric acid showed a strong difference, with respect to the ones prepared with the same precursors but with hydrochloric acid, in their virucidal effect upon irradiation with visible light.

In particular, the virucidal activity of the different CDs under visible or ultraviolet light exposure has been evaluated against SARS-CoV-2 (Human 2019-nCoV strain INMI1, from Istituto "L. Spallanzani", Rome, Italy) according to guidelines reported in EN14476 for testing of chemical disinfectants.

Therefore, object of the invention is:

- a process for the preparation of carbon dots comprising

  a. dissolving Glycine, 1,5-diaminonaphthalene and boric or hydrochloric acid in distilled water,
  b. sonicating the mixture obtained in a. until the solution becomes turbid gray
  c. submitting the turbid grey solution thus obtained to thermal treatment and solubilising the product thereby obtained in distilled water
  d. filtering the mixture obtained in c. with a 10-30 $\mu$m pore size filter and collecting the eluate
  e. drying the eluate thus obtained

  wherein Glycine, 1,5-diaminonaphthalene and boric or hydrochloric acid and water are the sole compounds used in said process;
- carbon dots obtainable by the process for the preparation of carbon dots in any of the embodiments disclosed herein and claimed;
- a composition comprising the carbon dots as herein disclosed and claimed and at least one carrier and/or excipient;
- a product coated or loaded with the carbon dots or with the composition as herein disclosed and claimed,
- a medical device comprising the carbon dots or with the composition as herein disclosed and claimed,
- the carbon dots or with the composition as herein disclosed and claimed for use in a medical treatment,
- the use of carbon dots or with the composition as herein disclosed and claimed for the disinfection, sanitisation and/or sterilisation of surfaces, objects, air, waters, areas.
- a method for the disinfection, sanitisation and/or sterilisation of surfaces, objects, air, waters, areas wherein

comprising
putting in contact the object of the disinfection, sanitisation and/or sterilisation with a suitable amount of the carbon dots or with the composition as herein disclosed and claimed and subjecting it to irradiation with visible and/or UV light for a period of time of at least 5 minutes,

- a medical treatment for the treatment of infectious diseases wherein the carbon dots or with the pharmaceutical composition as herein disclosed and claimed are administered to a patient in need thereof and said carbon dots or with the composition as herein disclosed and claimed are irradiated or exposed to UV light and/or visible light;
- a medical treatment of a plant, wherein said plant or parts thereof are treated with the carbon dots or with the composition as herein disclosed and claimed, and said plant or parts thereof is exposed to visible light.

According to the invention, the sole compounds used in the process herein disclosed and claimed are Glycine, 1,5-diaminonaphthalene distilled water and one of boric or hydrochloric acid.

GLOSSARY

In the present description UV, or UV light has the meaning commonly intended in the art, i.e. it is a form of electromagnetic radiation with wavelength from 10 nm (with a corresponding frequency around 30 PHz) to 400 nm.

In the present description, visible light has the meaning commonly intended in the art, i.e. visible light spectrum is the segment of the electromagnetic spectrum that the human eye can view. More simply, this range of wavelengths is called visible light. Typically, the human eye can detect wavelengths from about 400 to 700 nanometers.

Carbon quantum dots (CQDs, C-dots or CDs) are small carbon nanoparticles (less than 10 nm in size), they have been discovered in 2004 and are a new class of fluorescent carbon nanomaterials. CQDs possess the attractive properties of high stability, good conductivity, low toxicity, environmental friendliness, simple synthetic routes as well as comparable optical properties to quantum dots.

In the present description the term irradiated with (e.g. UV and/or visible light) can be substituted in any part with the term exposed to (e.g. UV and/or visible light).

The term Glycine in the present invention refers to the non-functionalised amino acid Glycine.

Gly-DiaN in the present description refers to carbon dots prepared as described in the example "Synthesis of Gly-DiaN, DiaN-CA and Gly-DiaN-BA" in the section example below, from Glycine 1,5-diaminonaphthalene, HCl dissolved in distilled water DiaN-CA in the present description refers to carbon dots prepared as described in the example "Synthesis of Gly-DiaN, DiaN-CA and Gly-DiaN-BA" in the section example below, from Citric Acid, 1,5-diaminonaphthalene and HCl dissolved in distilled water. Gly-DiaN-BA in the present description refers to carbon dots prepared as described in the example "Synthesis of Gly-DiaN, DiaN-CA and Gly-DiaN-BA" in the section example below, from Glycine, 1,5-diaminonaphthalene and boric acid dissolved in distilled water.

The term antimicrobial in the present description can be intended as a compound having virucidal and/or bactericidal and/or fungicidal activities or a mixture thereof, or to said activities itself. Antimicrobial according to the invention can also refer to a compound having phytoplasmicidal activities of to the activity itself.

DETAILED DESCRIPITION OF THE FIGURES

Figure 1. Experimental setup. Drops of viral suspension and nanomaterials were put on a 96-well plate lid for the different treatments (exposure to visible light, ultraviolet light or left in the dark).

Figure 2 A, B and C, shows the UV-Vis spectra of the 3 samples featured by a main absorption band. The absorption band peaked at 320 nm is attributed to the n $\Rightarrow \pi^*$ transitions of the carbonyl and amide bonds (C=O and C=N) [1].

Figure 3. Excitation-emission-intensity spectra of the Gly-DiaN, DiaN-CA and Gly-Dian-BA, in water solution (a,b and c respectively). The photoluminescence intensity is reported in false color scale from blue to red.

The figure shows 3D excitation-emission-intensity maps of samples A) Gly-DiaN, B) DiaN-CA and C) Gly-Dian-BA carbon dots respectively. Panels A and B exhibit a maximum of photoluminescence intensity at 454 nm of emission and 360 nm of excitation. In contrast, the sample C shows a maximum of photoluminescence emission peaked at 404 nm when excited at 350 nm. With respect to the CDs photoluminescence, the 3 systems show a narrower excitation range. Previously the excitation-dependent emission of CDS has been attributed to a wide variety of emitting centers in the nanoparticles, which are formed with the uncontrolled reactions of the carbonization processes. Following this assumption, a narrow excitation range could indicate a particular type of CD, with a more controlled chemical composition and structure.

Figure 4. A, B and C FTIR spectra, respectivelty, of Gly-DiaN, DiaN-CA and Gly-DiaN-BA in the 4000-400 cm-1 range. 4D Spectrum of the Gly-DiaN-BA sample in the 3700-2500 cm-1 range in comparison with that of boric acid used as the reference.

The bands at 3005 and 2823 cm-1 are attributed to the C-H bond stretching while the band at 1413 cm-1 to the CO-NH amide group. A significative difference between the Gly-DiaN-BA 4C and the other two samples (4A and 4B) is the presence of boric acid. This is accountable for the band at 3215 cm-1 assigned to the O-H stretching of the acid (figure 4D).

Figure 5. FTIR spectra of Gly-DiaN (5A and 5B) and Gly-DiaN-BA (5C and 5D) after thermal treatment at 150 and 250 °C for 2 h.

As previously observed, the presence of boric acid catalyzes the polymerization of the reagents at low temperatures. The FTIR spectra of Figure 5 show the difference between Gly-DiaN-BA and Gly-DiaN when treated at 150 and 250 °C respectively, for 2 hours.

Figure 6. EPR spectra of the Gly-DiaN, DiaN-CA and Gly-DiaN-BA collected in dark conditions (a,c and e) and under UV irradiation (b, d and f).

EPR spectra obtained from samples in powders (Figure 6) show that all the 3 systems produce radical species, both in the dark and in presence of UV irradiation of UV irradiation.

Figure 7. TGA (black line) and DTA (dotted line) analysis from 25 °C to 500 °C of the Gly-DiaN, DiaN-CA and Gly-DiaN-BA samples (a, b and c respectively).

The TG-DTA analysis in Figure 7 shows the thermal decomposition of a) Gly-DiaN, b) DiaN-CA and c) Gly-DiaN-BA.

Figure 8. Virucidal effect of carbon dots Gly-DiaN, DiaN-CA and Gly-DiaN-BA exposed to UV light (5 minutes at 365 nm) and to visible light (5 minutes at 450 nm).

#1 = Gly-DiaN
#2 = DiaN-CA
#3 = Gly-DiaN-BA

Figure 9. Fluorescence analysis in the range 300-700 nm

The emission of an aqueous solution of dots, having a concentration of 0.1 mg/mL, was evaluated in the wavelength range of 300-700 nm. The maximum of emission of Gly-DiaN is centered at 450 nm, of DiaN-CA at 445 nm and Gly-Dian-BA at 404 nm. The emission of each dot was measured at three different excitation wavelengths: 320, 340 and 360 nm. Fig. 9a Gly-DiaN, Fig. 9b DiaN-CA, Fig. 9c Gly-DiaN-Ba.

Figure 10. UV-Vis analysis in the range 200-600 nm

The absorbance of an aqueous solution of dots, having a concentration of 0.1 mg/mL, was measured in the wavelength range of 200-600 nm. The maximum peak of absorbance is between 310 nm and 340 nm and the maximum of the absorbance is centered at 330 nm.

The figure shows an absorbance higher than 0.5 in the maximum peak at the testing conditions used.

Figure 11. Stability over time

Stability over time of Gly-Dian-BA was evaluated over 40 days. The absorbance at 330 nm of an aqueous solution of dots, having a concentration of 0.1 mg/mL, was measured at time 0, after 7 days, after 31 days and after 40 days. As can be seen, the absorbance was consistent over the period.

Figure 12. Antiviral effect (SARS-CoV-2 virus Wuhan strain) of a surface functionalized with Gly-DiaN-BA.A volume of viral suspension (5 μL) of against SARS-CoV-2 virus (Wuhan strain) was casted on a 96-well plate lid (12x8 cm). Then the viral suspension was added to the coated plates.

The substrates were then left in the dark or exposed to ultraviolet (UV = 365 nm) or visible light (VL = 450 nm) for 5 minutes. After an additional 5-minute incubation, viral suspensions were collected and viral titer was determined according to the methodology described below.

Viral titre drops to 0 when the surface functionalised with Gly-DiaN-BA CDs is irradiated with UV and decreases visibly also when the surface functionalised with Gly-DiaN-BA CDs is exposed to visible light.

Figure 14 Untreated (14a) and treated (14b) slices from the same fruit (strawberry) were kept at room temperature under different conditions, changes in the slices were monitored over time.

The figure shows untreated slices with extensive mould growth and rottening after 3 days (14a) whereas treated slices (14b) show signs of drying but no mould or rottening after 196 days.

Figure 15 shows TEM analysis of Gly-DiaN and DiaN-CA.

Transmission electron microscopy images were obtained by using an FEI TECNAI 200 microscope working with a field emission electron gun operating at 200 kV. Sample preparation was done by dispersing the nanoparticles in ethanol by ultrasonication and then dropping them onto a carbon-coated copper grid and drying them for observations.

DETAILED DESCRIPTION OF THE INVENTION

The present invention relates to a process for the preparation of carbon dots comprising

a. dissolving Glycine, 1,5-diaminonaphthalene and boric or hydrochloric acid in distilled water,
b. sonicating the mixture obtained in a. until the solution becomes turbid gray
c. submitting the turbid grey solution thus obtained to thermal treatment and solubilising the product thereby obtained in distilled water
d. filtering the mixture obtained in c. with a 10-30 μm pore size filter and collecting the eluate
e. drying the eluate thus obtained

wherein Glycine, 1,5-diaminonaphthalene, water and either boric or hydrochloric acid are the sole compounds used in said process.

Hence, in one embodiment of the invention the process is carried out with the compounds Glycine, 1,5-diaminonaphthalene, water and boric acid and in a second embodiment the process is carried out with the compound Glycine, 1,5-diaminonaphthalene, water and hydrochloric acid.

No other compounds are used in the process of the invention, hence the mixture resulting from step a. consists of Glycine, 1,5-diaminonaphthalene and boric or hydrochloric acid dissolved in distilled water. No other reagents, catalysers, solvents are used in the whole process.

As indicated in the summary and demonstrated in the experimental part and in the figures, the presence of Boric or hydrochloric Acid in the process of the invention provides carbon dots with remarkable virucidal activity. In particular, the virucidal activity is further enhanced when in the same method and with the same amounts of Glycine and 1,5-diaminonaphthalene Boric acid is used instead of HCl. In particular, the carbon dots obtainable with the process herein disclosed and claimed show unexpected exceptional virucidal properties upon irradiation with visible light.

In an embodiment of the invention, when Boric acid is used in step a., the ratio (weight) of Glycine:1,5-diaminonaphtalene: boric acid used in a. is of about 1.0:2.0:0.8; preferably the ratio is of 1.0:2.06:0.8.

In other words, in the process of the invention the 100% in weight of said Glycine, 1,5-diaminonaphthalene and boric acid is thus composed:

Glycine 25-26% in weight
1,5-diaminonaphthalene 53-54% in weight
Boric acid 20-21% in weight.

In an embodiment, in step a. 0.15 g of Glycine, 0.31 g of 1,5-diaminonaphthalene and 0.12 g of boric acid are dissolved in 15-25 ml of distilled water, preferably in 20 ml of distilled water. A mixture of Glycine, 1,5-diaminonaphthalene, boric acid and water is thereby obtained. Multiples of the above can be used.

When hydrochloric acid is used in step a., the molar ratio of Glycine:1,5-diaminonaphtalene: Hydrochloric acid in step a. is of about 1.0:1.0:0.25-0.75; preferably the ratio is of 1.0:1.0:0.5.

In an embodiment, 0.15 g of Glycine, 0.31 g of 1,5- diaminonaphthalene and 0.0415 - 0.1245 mL of HCL 1M, preferably 0.083 mL, are dissolved in 20 ml of distilled water. As clear from the description and from the claims, boric acid and hydrochloric acid are mutually exclusive, therefore in step a. either one or the other acid will be used together with only Glycine, 1,5 diaminonaphthalene and water. Multiples of the above can be used.

According to the process of the invention, in b. the mixture obtained in a. is sonicated until it becomes turbid grey and then it is subjected to c. thermal treatment, and the product obtained after the thermal treatment is suspended in distilled water.

The thermal treatment in c. in any of the embodiment of the process for the preparation of carbon dots herein disclosed, can be carried out in any of the ways commonly used by the skilled person such as in microwave at suitable W-time balance, or in an oven at 200°C for a time range not shorter than 12 h or in a autoclave under hydrothermal conditions at 200°C for at least 8 h.

In a preferred embodiment, the thermal treatment is carried out for a time period of 2-5 minutes at 800-1200 W in a microwave oven, more preferably for a time period of about 2 minutes at 1000 W.

Once the thermal treatment has been carried out, the product thereby obtained suspended in distilled water.

Preferably, the amount of distilled water used in c. is similar to or the same, amount of distilled water used in a.

In d. the mixture obtained in c. is dried and the resulting carbon dots are in the form of a powder of a black-violet colour.

The drying procedure of the mixture can be carried out according to any drying procedure commonly used in protocols for the preparation of carbon dots. By way of example the mixture can be dried by heating for a suitable period of time or in air at room temperature for about 48h, or under vacuum for about 24 h.

A common drying procedure is to carry out said drying by submitting said eluate at a temperature of 50-70°C for a period of time of 12-18 hours.

The present invention hence relates to carbon dots obtainable with the process of disclosed above and in the claims.

As shown in the experimental examples below and in the figures, the carbon dots of the invention show a strong antiviral effect when irradiated with UV and/or visible light.

The carbon dots prepared according to the method, indicated also in the present description and figures as Gly-DiaN-BA of the invention have been compared to carbon dots prepared with a process as disclosed above wherein in step a. the boric acid catalyst was substituted with the catalyst HCl and to carbon dots prepared (defined in the present description and figures as Gly-DiaN) and to carbon dots prepared with a process as disclosed above wherein in step a. no catalyst was used and Citric acid was used instead of Glycine in a w:w ratio Citric acid: 1,5-diaminonaphthalene of 1:0.81 (defined in the present description and figures as DiaN-CA). The CDs prepared according to the process of the invention Gly-DiaN-BA and Gly-DiaN show unexpected and non-predictable improved antiviral properties when compared to CDs DiaN-CA , in particular, Gly-DiaN-BA shows further improved antiviral properties when compared to CDs Gly-DiaN and DiaN-CA.

The CDs of the invention as well as the comparative CDs have been characterised as disclosed in figures 2-8, 9, 10 and 15.

Ultraviolet-visible (UV-vis) spectra of the samples solubilized in water were measured in absorbance mode from 200 to 600 nm by using a "Nicolet Evolution 300" UV-vis spectrophotometer (Thermo Fisher) Figure 2.

Fourier-transform infrared (FTIR) analysis of the samples was carried out by a "Vertex 70" interferometer (Bruker) in the range of 4000-400 cm-1 with a 4 cm-1 resolution, Figure 4 and 5.

Fluorescence analysis of the samples solubilized in water was carried out by a "Horiba Jobin Yvon NanoLog" spectrofluorometer with an excitation and emission wavelength from 300 to 700 nm.

Transmission electron microscopy analysis was carried out by a "Fei Technai G2 F20 Twin TMP", working with a field emission electron gun operating at 200 kV.

Thermal gravimetric analysis (TGA) was measured from room temperature to 500 °C with 10 °C min-1 of ramp rate under nitrogen flow of 20 mL min-1 by an SDT-Q600 instrument (TA Instruments), Figure 7.

TEM characterization of the CDs of the invention and of the control CDs was carried out, all CDs resulted to have an average size around 5-6 nm, no evidence of crystalline structure was observed.

Fluorescence analysis in the range of 300 nm and 700 nm was carried out. Measurements were carried out at a CDs concentration of 0.1 mg/ml in water. The emission of an aqueous solution of Gly-DiaN, DiaN-CA and Gly-DiaN-BA CDs, each solution having a concentration of 0.1 mg/mL, was evaluated in the wavelength range of 300-700 nm as reported in figure 9. The maximum emission peak observed for Gly-DiaN is at excitation wavelength of 360 nm and the peak is between 425 and 475, the maximum of emission of being centered at 450 nm (Figure 9a); the maximum emission peak observed for DiaN-CA is at excitation wavelength of 360 nm and the peak is between 425 and 475, the maximum of emission being centered at 445 nm (Figure 9b); and the maximum emission peak observed for Gly-Dian-BA is at excitation wavelength of 320 nm and the peak is between 375nm and 425 nm, the maximum emission being centered at 404 nm (Figure 9c). The emission of each dot was measured at three different excitation wavelengths: 320, 340 and 360 nm. The data obtained show that at the measured wavelengths no shift of the peak is observed.

Gly-DiaN-BA emission peak shows an intensity/a.u. higher than $1.6x10^7$, up to about $1.8x10^7$ between 357 nm and 425 nm upon excitation at 320 nm.

UV.vis analysis was also carried out in the range from 200 nm to 600 nm for Gly-DiaN, DiaN-CA and Gly-DiaN-BA CDs as reported in Figure 10. The absorbance of an aqueous solution of dots, having a concentration of 0.1 mg/mL, was measured in the wavelength range of 200-600 nm. For all samples tested the The maximum of the absorbance is centered at 330 nm.

All CDs show a maximum peak of adsorbance between 310 nm and 340 nm, the peak being centered at about 330 nm.

Figure 11 reports the stability over time of Gly-DiaN-BA.

TEM analysis is showed in figure 15.

Once characterised, the cytotoxicity of the CDs of the invention and the control CDs was assessed and their virucidal efficacy with UV light and with visible light (Figure, 8, 12 and 13) was analysed in various experiments. The virucidal activity of the CDs of the invention and the control CDs was assessed on SARS-Cov2 (various strains) infected Vero E6 cells (kidney epithelial cells from African green monkey, ATCC CRL-1586) and the results shown in figure 8 12 and 13, and in the tables in the experimental section below show that while the virucidal activity of the CDs of the invention Gly-DiaN-BA and CDs Gly-DiaN is comparable after irradiation with UV light (0% viral titer), whereas only Gly-DiaN-BA CDs of the invention show an equal virucidal activity after irradiation with visible light (0% viral titer) whereas CDs Gly-DiaN show a lower virucidal activity (30% viral titer). CDs DiaN-CA show extremely low virucidal activity (63% viral titer) after irradiation with visible light.

Therefore, the CDs of the invention show a unexpected advantageous property and, in particular, Gly-DiaN-BA shows further unexpected advantage with respect to Gly-DiaN when the virucidal effect is tested in visible light.

A further object of the invention is a composition comprising the carbon dots of the invention and at least one carrier and/or excipient.

Depending on the final form of the composition, the skilled person will easily select suitable carrier/s and/or excipient/s.

This composition can be by way of example in the form of a gel, of a solution, of an emulsion, of a spray, of an aerosol, of a powder.

According to an embodiment of the invention, the composition can be a sanitizing or a cleaning composition, a paint, a varnish, a gloss, a dye. In an embodiment, the composition can be loaded onto an adsorbent substrate in order to form a sanitizing wipe or a sanitizing filter or the like. The composition can be also loaded in a dispensing device comprising a reservoir for the storage thereof and means for the release, preferably in a dose controlled and even more preferably also in a time controlled manner so to be released in the air or in a fluid in a pre-defined amount in one or more period of time. In a further embodiment, the sanitizing or cleaning composition can be of food grade quality.

In a further embodiment said carrier/s and/or excipient/s are suitable for the administration of the composition on a plant or on selected parts of a plant thereby allowing the treatment of plant infectious diseases such as diseases caused by plant viruses, or plant bacterial diseases or plant fungal diseases or plant diseases caused by phytoplasmas.

When plants are treated, the infected part of the plant can be, by way of example, sprinkled or sprayed on the affected plant parts and the plant is then exposed to visible light if not already placed under visible light during and after the treatment.

Therefore, the invention also relates to a medical treatment of a plant, wherein said plant or parts thereof are treated with the carbon dots or with the composition as herein disclosed and claimed and said plant or parts thereof is exposed to visible light.

In a preferred embodiment said suitable carrier/s and/or excipient/s are of pharmaceutical grade (i.e. pharmaceutically acceptable) and the composition is a pharmaceutical composition.

The pharmaceutical composition of the invention can be a composition suitable for oral, parenteral, subcutaneous, endovenous, inhalatory, topical, rectal, intravaginal administration.

Hence, accordingly, the carbon dots or composition as herein defined and claimed can be administered orally, systemically, parenterally, by injection, by endovenous injection, by aerosol, by nebulization, topically, intranasally, nosepharingeally and/or oropharingeally, rectally, intravaginally.

The pharmaceutical composition can hence be in the form of a powder, of granules, of a solution, of an emulsion, of a cream, of a gel, of an ointment, of a spray, of an aerosol, of a suspension, of a syrup, of an injectable fluid or the like.

The person skilled in pharmaceutical preparation will readily identify suitable carriers and/or excipients in order to prepare the composition of the invention.

The composition can also be provided already aliquoted in single dosages or in single dosage fractions, or can be provided in a dispenser apt to release single dosage or single dosages fractions, such as, by way of example, a dispenser apt to release a pre-defined amount of a solution in the form of a spray.

An object of the invention is also a product coated with the carbon dots or with the composition according to any of the embodiments herein disclosed or as defined in the claims.

Without being limited thereto, said product can be a sanitizing or cleaning product, a medical device, a medical equipment, an individual protection device, a system for air filtration, a fabric, a medicated bandage, a medicated patch, a surface, an object.

In a preferred embodiment, medicated bandages or medicated patches will be UV and/or visible light transparent thereby allowing the carbon dots of the invention to exert their antimicrobial activity.

Exposure, even to mere visible light, of each and any of said products will result in the activation of the carbon dots of the invention thereby triggering their antimicrobial activities.

The invention also relates to a medical device comprising/loaded with the carbon dots the carbon dots or the composition according to any of the embodiments herein disclosed or as defined in the claims. In a preferred embodiment, said medical device is a drug delivery device and hence comprises a reservoir for the storage of said carbon dots or composition and means for their release, in a dose controlled way or in a dose controllable way. The drug delivery device can be a self-administering device or a surgical device (e.g. a laparoscopic device).

Preferably the device will further comprise means for the emission UV and/or visible light for a desired period of time.

By way of example, the device can be a device for nasopharyngeal delivery, and it can be equipped with a light emitting portion designed so as to produce a light when the drug is administered, in order to activate, in situ, the carbon dots of the invention and allowing them to exert their antimicrobial activity.

In other embodiments, when the device is a surgical device for laparoscopy, the device can be equipped with a light emitting portion or, the light suitable for the activation of the carbon dots of the invention can be emitted by an additional device as commonly used in laparoscopy. The light can be UV and/or visible light.

Given the extremely short amount of time necessary for the activation of the antimicrobial activity of the carbon dots of the invention, they, or pharmaceutical composition comprising them, are extremely suitable for laparoscopic surgery, both for the prevention of infections deriving from the surgery itself, as their activation would kill microbial

infectious agents potentially introduced by the laparoscopy itself, as well as for acting on localised internal infections thereby avoiding or strongly limiting the use of antibiotics and/or antiviral drugs.

An object of the invention is hence represented by the carbon dots or the composition according to any of the embodiments herein disclosed or as defined in the claims for use in a medical treatment.

The medical treatment of the invention comprises the administration of the carbon dots or with the pharmaceutical composition as herein disclosed and claimed to a patient in need thereof and said carbon dots or with the composition as herein disclosed and claimed are irradiated or exposed to UV light and/or visible light.

According to an embodiment of the invention the carbon dots or with the pharmaceutical composition as herein disclosed and claimed can be used in the treatment of a microbial infection or as adjuvant in a treatment of a microbial infection or in a treatment for the prevention of a microbial infection or of an infectious disease.

Due to the observed antiviral activity, the carbon dots or the composition of the invention in any of the embodiments provided in the present description can be used in the treatment or prevention, or in adjuvating the treatment or the prevention of bacteria, fungal or viral infections.

The antiviral activity triggered by the simple visible light demonstrated in the examples below supports the preventive effect as well as the therapeutic effect of the carbon dots and composition of the invention.

The invention therefore also encompasses a method for the treatment or prevention, or for adjuvating the treatment or the prevention of bacterial, fungal or viral infections or bacterial, fungal or viral diseases wherein therapeutically effective doses of the carbon dots or of the composition described hereinabove are administered at a therapeutically effective dosage to a subject in need thereof and irradiated (exposed to) with UV or visible light.

The administration can be repeated several times per die and the carbon dots or the composition can be supplied already in the form of single dosages or with a single-dose dispenser.

Therapeutically effective amount means an amount that is effective in therapy, or an amount sufficient to provide a desired therapeutic effect. An amount that is effective in therapy is an amount which produces one or more desired biological activities during the treatment or the prevention of a disease.

As described above in the part related to medical/surgical devices, the carbon dots or composition of the invention can be administered via a laparoscopic surgery which is a treatment that is usually carried out in the presence of light emitting tools.

In a preferred embodiment, the medical treatment is a treatment or the prevention of a viral infectious disease as defined herein.

According to an embodiment said virus is an animal DNA virus or an animal RNA virus. The viral disease can be an animal disease wherein said animal is a mammal, a fish, a reptile, a bird.

In particular the animal can be a human, a horse, a pig, a bird, a small ruminant (such as goats, sheep and other wild small ruminants, a large ruminant (such as a bovine, an equine, or other wild large ruminants), a rodent, dog, a cat, a primate, a feline, a small or a large bird, chicken, a fish, an insect.

By way of example, the insect can be a bee, and the carbon dots of the invention can be used to treat bees' viral infections.

According to a preferred embodiment said animal is a human.

By way of example the disease can be African horse sickness (ASH), African swine fever (ASF), Avian influenza (AIV), Bluetongue (BT), Crimean-Congo Haemorrhagic fever (CCHF), Foot and mouth disease (FMD), Newcastle disease (ND), Peste des petits ruminants (PPPR), Porcine Epidemic Diarrhoea (PED), Rift Valley fever (RFV), Rinderpest (RPV), Schmallenberg virus (SBV), Schmallenberg virus (SBV), Viral diseases in aquaculture, West Nile Virus (WNV), Canine distemper, Canine influenza, Canine parvovirus, Rabies, Canine Coronavirus gastroenteritis, feline immunodeficiency virus (FIV), Feline Leukaemia Virus (FeIV), Feline coronavirus, Feline Infectious Peritonitis (FIP), Feline Parvovirus, Hepatitis, Haemorrhagic fever (Ebola), Encephalitis, Mononucleosis, Covid-19, MERS, Herpesvirus lesions, HIV, Varicella, Gastroenteritis and others.

A non-limiting example of suitable animal viruses is provided in the table below.

**Families of Viruses That Infect Humans**

| Family Name | Genome | Capsid Morphology | Envelope |
|---|---|---|---|
| **Adenoviridae** | dsDNA | Icosahedral | Naked |
| **Anelloviridae** | ssDNA(−)[f] | Icosahedral | Naked |
| **Arenaviridae** | ssRNA(−)[g,h] | Helical | Enveloped |
| **Astroviridae** | ssRNA(+)[i] | Icosahedral | Naked |
| **Bornaviridae**[a] | ssRNA(−)[g] | Helical | Enveloped |
| **Bunyaviridae** | ssRNA(−)[g,h] | Helical | Enveloped |
| **Caliciviridae** | ssRNA(+)[i] | Icosahedral | Naked |
| **Coronaviridae**[b] | ssRNA(+)[i] | Helical | Enveloped |
| **Filoviridae**[a] | ssRNA(−)[g] | Helical | Enveloped |
| **Flaviviridae** | ssRNA(+)[i] | Icosahedral | Enveloped |
| **Hepadnaviridae** | Partially ssDNA | Icosahedral | Enveloped |
| **Hepeviridae** | ssRNA(+)[i] | Icosahedral | Naked |
| **Herpesviridae**[c] | dsDNA | Icosahedral | Enveloped |
| **Orthomyxoviridae** | ssRNA(−)[g] | Helical | Enveloped |
| **Papillomaviridae** | dsDNA | Icosahedral | Naked |
| **Paramyxoviridae**[a] | ssRNA(−)[g] | Helical | Enveloped |
| **Parvoviridae** | ssDNA | Icosahedral | Naked |
| **Picobirnaviridae** | dsRNA | Icosahedral | Naked |
| **Picobirna** | dsRNA | Icosahedral | Naked |
| **Picornaviridae**[d] | ssRNA(+)[i] | Icosahedral | Naked |
| **Pneumoviridae** | ssRNA(−)[g] | Helical | Enveloped |
| **Polyomaviridae** | dsDNA | Icosahedral | Naked |
| **Poxviridae** | dsDNA | Complex | Varies |
| **Reoviridae** | dsRNA | Icosahedral | Naked |
| **Retroviridae** | ssRNA(+)[i] | Complex | Enveloped |
| **Rhabdoviridae**[a] | ssRNA(−)[g] | Helical | Enveloped |
| **Togaviridae** | ssRNA(+)[i] | Icosahedral | Enveloped |
| **Delta**[e] | ssRNA(−)[g] | Icosahedral | Enveloped |

[a]Mononegavirales.
[b]Nidovirales.
[c]Herpesvirales.
[d]Picornavirales.
[e]Floating genus, which is not currently assigned to a viral family. It bears some similarities to viroid pathogens of plants.
[f]ssDNA(+) indicates the mRNA-coding strand.
[g]ssRNA(−) indicates the complement of the message-sense strand.
[h]Some segments are ambisense.
[i]ssRNA(+) indicates a message-sense strand.
ds, double stranded; ss, single stranded.

From table 201.2 of "Classification of Human Viruses" R,D, Siegel; Principles and Practice of Pediatric Infectious Diseases. 2018 : 1044-1048.e1

In a further embodiment, the virus is an animal or human Coronavirus, Orthomyxovirus, Filovirus, Flavivirus, Hepadnavirus, Hepevirus, Herpesvirus, Papillomavirus, Pneumovirus, Poxivirus, Rhinovirus, Reovirus and/or Togavirus.

In a particularly preferred embodiment said Coronavirus is one of SARS, MERS or SARS-CoV-2, FCoV.

According to the invention, the carbon dots or composition for can be administered orally, systemically, parenterally, by injection, by aerosol, by nebulization, topically, intranasally, nasopharyngeal and/or oropharyngeal, rectally, intravaginally.

Object of the invention is also the use of the carbon dots or of the composition as herein defined and claimed for the disinfection, sanitisation and/or sterilisation of surfaces, objects, air, waters, areas.

By way of example, they can be used for the treatment of water such as drinking water and water for animal breeding, by mixing said waters or treating said waters with said carbon dots or with said composition and submitting said water to visible and/or UV light.

Therefore, the invention also relates to a method for the disinfection, sanitisation and/or sterilisation of surfaces, objects, air, waters, areas wherein comprising

putting in contact the object of the disinfection, sanitisation and/or sterilisation with a suitable amount of the carbon dots as defined or of the composition as defined in the present description and in the claims and subjecting it to irradiation with or exposure to visible and/or UV light for a period of time of at least 5 minutes.

All the experiments in the examples below were carried out on the commercially available cell Line Vero E6.

In any part of the present description and claims the term comprising can be substituted by the term "consisting of".

All the examples were carried out with the nanoparticles comprising a L-Lysine (hyper)branched nanopolymer prepared according to example 7 indicated also as "lysine-B nanomaterial".

It is herein declared that all experiments involving cells were carried out on commercially available African green monkey Vero E6 cells.

EXAMPLES

**Synthesis of Gly-DiaN, DiaN-CA and Gly-DiaN-BA**

To obtain CDs, 3 different solutions containing different precursors were prepared: glycine and 1,5-diaminonaphthalene (Gly-DiaN), 1,5-diaminonaphthalene and citric acid (DiaN-CA) and glycine, 1,5-diaminonaphthalene and boric acid (Gly-DiaN-BA).

- Gly-DiaN: 0.15 g of Glycine and 0.31 g of 1,5-diaminonaphthalene and 0.83 mL of HCl 1 M were dissolved in distilled water (20 mL).
- DiaN-CA: 0.38 g of Citric Acid and 0.31 g of 1,5-diaminonaphthalene and 0.83 mL of HCl 1 M were dissolved in distilled water (20 mL).
- Gly-DiaN-BA: 0.15 g of Glycine, 0.31 g of 1,5-diaminonaphthalene and 0.12 g of boric acid were dissolved in distilled water (20 mL).

The solutions were sonicated until they became turbid gray and then treated at 1000 W for 2 minutes in a microwave oven. The obtained products were solubilized in 20 mL of distilled water, filtered with a 0.22 μm pore size filter and dried in the oven at 60°C for 15h to obtain black-violet powders.

The carbon dots obtained from 0.15 g of Glycine and 0.31 g of 1,5-diaminonaphthalene are denominated, in the present description and drawings as Gly-DiaN.

The carbon dots obtained from 0.38 g of Citric Acid and 0.31 g of 1,5-diaminonaphthalene are denominated, in the present description and drawings as DiaN-CA.

The carbon dots obtained from 00.15 g of Glycine, 0.31 g of 1,5-diaminonaphthalene and 0.12 g of boric acid are denominated, in the present description and drawings as Gly-DiaN-BA.

**Materials characterization**

Ultraviolet-visible (UV-vis) spectra of each of the carbon dots samples Gly-DiaN, DiaN-CA and Gly-DiaN-BA solubilized in water were measured in absorbance mode from 200 to 600 nm by using a "Nicolet Evolution 300" UV-vis spectrophotometer (Thermo Fisher).

Fourier-transform infrared (FTIR) analysis of the samples was carried out by a "Vertex 70" interferometer (Bruker) in the range of 4000-400 cm-1 with a 4 cm-1 resolution.

Fluorescence analysis of the samples solubilized in water was carried out by a "Horiba Jobin Yvon NanoLog" spectrofluorometer with an excitation and emission wavelength from 300 to 700 nm.

Transmission electron microscopy analysis was carried out by a "Fei Technai G2 F20 Twin TMP", working with a field emission electron gun operating at 200 kV.

Thermal gravimetric analysis (TGA) was measured from room temperature to 500 °C with 10 °C min-1 of ramp rate under nitrogen flow of 20 mL min-1 by an SDT-Q600 instrument (TA Instruments).
The results are provided in figures 2-7.

**Cytotoxicity analysis**

Cytotoxicity of each of the carbon dots samples Gly-DiaN, DiaN-CA and Gly-DiaN-BA was studied in Vero E6 cells (Cercopithecus aethiops, kidney, ATCC CRL-1586). Cell line is routinely maintained in DMEM supplemented with 1% glutamine, 1% penicillin/streptomycin and 10% foetal bovine serum.
Exponentially growing Vero E6 cells were seeded into a 96-well plate at their optimal density in complete medium, 24 hours later cells were exposed to different concentrations of nanomaterials for 72 hours. Replicates for each concentration point were examined. Cytotoxic effect was evaluated through microscopy observation (determination of cell monolayer integrity). Drug dilutions were performed in culture medium. A cytotoxic concentration 50% (CC50, concentration resulting in 50% loss of cell viability compared to untreated control) was calculated.
The following table shows the effect of nanomaterials on cell viability in terms of cell monolayer integrity after 72 hours of treatment at the indicated concentrations.
For carbon dots #1 and #3 the highest concentration that could be tested was 5 mg/mL due to water solubility constraints, therefore the cytotoxic concentration 50% (CC50) was calculated through interpolation of the dose-response curves. For #2 it was possible to reach 25 mg/mL

**Table 1.** Cytotoxicity of nanomaterials. Effect of nanomaterials on cell monolayer integrity.

| | **Cell monolayer integrity (viability)** | | |
|---|---|---|---|
| **Test material** | **5 mg/mL** | **0.5 mg/mL** | **0.05 mg/mL** |
| Carbon dots #1 | 0 | 100 | 100 |
| Carbon dots #3 | 0 | 100 | 100 |
| | | | |
| **Test material** | **25 mg/mL** | **2.5 mg/mL** | **0.25 mg/mL** |
| Carbon dots #2 | 100 | 100 | 100 |
| #1 = Glycine + 1,5-diaminonaphtalene + HCl<br>#2 = Citric Acid + 1,5-diaminonaphtalene + HCl<br>#3 = Glycine + 1,5-diaminonaphtalene + Boric Acid<br>#1 and #3 had a $CC_{50}$ of 2.5 mg/mL.<br>#2 had a $CC_{50}$ of >25 mg/mL. | | | |

**Virucidal activity determination**

Virucidal quantitative suspension test for chemical disinfectants and antiseptics in the medical area has been performed, according to guidelines in EN 14476:2013+A2:2019/UNI EN 14476:2019.
A volume of viral suspension (as indicated for each experiment below in results section) of SARS-CoV-2 (Wuhan strain) has been put on a 96-well plate lid (12x8 cm). A volume of nanomaterial has been added to the viral suspension (at the indicated volume and final concentrations below in results section).
Suspensions were then left in the dark or exposed to visible (450 nm) or ultraviolet light (365 nm) for the time indicated in each experiment (range 2-5 minutes).
Culture medium was used as control, added to the viral suspension and exposed to the dark or to visible/ultraviolet light as with nanomaterials. General experimental setup is shown in Figure 1.
After an additional 5-minute incubation, viral suspensions were collected and viral titer was determined according to the methodology described below.

**Viral titer determination**

An in vitro system was employed to determine viral titer of SARS-CoV-2 suspensions.
Vero E6 cells (kidney epithelial cells from African green monkey, ATCC CRL-1586) were maintained at their optimal density based on ATCC data sheet. On Day 1 of the experiment cells were transferred in 96-well plate (20.000 cells per well). On Day 2 cells were infected with the serial viral dilutions (10-2, 10-3, 10-4...) in 6-well replicates for each

condition. Importantly, treatment concentration of each nanomaterial was determined in order to get non-cytotoxic concentration at the first serial dilution added to cells.

After 3 additional days infection in each test well was determined by observation of the cytopathic effect. Data were then used to determine the titer of treated and control virus according to Reed and Muench method (Reed, L.J.; Muench, H. (1938). "A simple method of estimating fifty percent endpoints". The American Journal of Hygiene. 27: 493-49):

Infection data were employed to build the following table:

| A | B | C | D | E | F |
|---|---|---|---|---|---|
| Viral dilution | Number of affected wells | Number of unaffected wells | Cumulative number of affected wells | Cumulative number of unaffected wells | Affected ratio (%) |
| $10^{-2}$ | | | | | |
| $10^{-3}$ | | | | | |
| $10^{-4}$ | | | | | |
| $10^{-5}$ | | | | | |
| ... | | | | | |

Virus titer determination:

Put in the first row the virus dilution with 6/6 affected wells and in the last row that one with 0/6 affected.

Write in column B the number of affected wells and in column C the number of unaffected wells.

Column D represents the cumulative number of affected wells calculated by adding numbers starting at the bottom of column B and adding up.

Column E represents the cumulative number of unaffected wells calculated by adding numbers starting at the top of column C and adding down.

Column F represents the cumulative ratio of affected wells (%): number from column D divided by the total of column D + E and express as %.

Calculate the proportionate distance between the two dilutions nearest to 50% using value from column F:

$$\frac{(\%\text{ affected next above }50\%) - 50\%}{(\%\text{ affected next above }50\%) - (\%\text{ affected next below }50\%)}$$

Add the negative exponent of the viral dilution next above 50%.

Use the calculated value as exponent with base 10 to obtain the virus titer within the infection volume (i.e., TCID50/100 μL).

**Virucidal efficacy of nanomaterials with UV and visible light**

Treatment concentration for each nanomaterial was selected in order to get a non-cytotoxic concentration at the first serial dilution added to cells during viral titer determination experiments. The following tables and related figures show virucidal effect of nanomaterials in presence or absence of either visible (450 nm) or ultraviolet light (365 nm) in different experiments.

Control = Cells infected with the virus
#1 = Glycine + 1,5-diaminonaphtalene + HCl
#2 = Citric Acid + 1,5-diaminonaphtalene + HCl
#3 = Glycine + 1,5-diaminonaphtalene + Boric Acid

**UV light exposure**

Experimental parameters:

5 µL viral suspension + 10 µL carbon dots (#1, #2 and #3 at 3.3 mg/mL).
Condition: UV light 365 nm (or dark)
Exposure time: 5 minutes light ON (or dark) + 5 minutes incubation

**Table 2.** Virucidal effect of carbon dots #1, #2 and #3 exposed to UV light.

| Test material | Viral titer ($TCID_{50}$/mL) |
|---|---|
| Control | 2,85E+05 |
| UV | 3,16E+05 |
| | |
| Carbon dots #1 | 2,31E+05 |
| Carbon dots #1 + UV | 0,00E+00 (<3.16E+02) |
| | |
| Carbon dots #2 | 4,33E+05 |
| Carbon dots #2 + UV | 2,31E+03 |
| | |
| Carbon dots #3 | 7,50E+04 |
| Carbon dots #3 + UV | 0,00E+00 (<3.16E+02) |

The results are depicted in figure 8.

**Visible light exposure**

Experimental parameters:

5 µL viral suspension + 10 µL carbon dots (#1, #2 and #3 at 3.3 mg/mL).
Condition: Visible light (VL) 450 nm (or dark)
Exposure time: 5 minutes light ON (or dark) + 5 minutes incubation

**Table 3.** Virucidal effect of carbon dots #1 and #3 exposed to visible light (VL).

| Test material | Viral titer ($TCID_{50}$/mL) |
|---|---|
| Control | 3,16E+05 |
| VL | 3,16E+05 |
| | |
| Carbon dots #1 | 3,16E+05 |
| Carbon dots #1 + VL | 1,43E+05 |
| | |
| Carbon dots #3 | 1,78E+06 |
| Carbon dots #3 + VL | 1,00E+04 |
| Carbon dots #2 | 3,98E+05 |
| Carbon dots #2 + VL | 2,51E+05 |

The results are depicted in figure 8.

**Antiviral effect (SARS-CoV-2 virus Wuhan strain) of a surface functionalized with Gly-DiaN-BA CDs**

Surface functionalization

Silicon substrates were at first coated with a hybrid silica film bearing amino groups via sol-gel process, and then functionalized with Gly-Dian-BA through an EDC/NHS coupling reaction.

Experimental parameters:

A volume of viral suspension (5 μL) of against SARS-CoV-2 virus (Wuhan strain) was casted on a 96-well plate lid (12x8 cm). Then the viral suspension was added to the coated plates.

The substrates were then left in the dark or exposed to ultraviolet (UV = 365 nm) or visible light (VL = 450 nm) for 5 minutes. After an additional 5-minute incubation, viral suspensions were collected and viral titer was determined according to the methodology described below.

Viral titer determination

An in vitro system was employed to determine viral titer of SARS-CoV-2 suspensions.

Vero E6 cells (kidney epithelial cells from African green monkey, ATCC CRL-1586) were maintained at their optimal density based on ATCC data sheet. On Day 1 of the experiment cells were transferred in 96-well plate (20.000 cells per well). On Day 2 cells were infected with the serial viral dilutions (10-2, 10-3, 10-4...) in 6-well replicates for each condition. Importantly, treatment concentration of each nanomaterial was determined in order to get non-cytotoxic concentration at the first serial dilution added to cells.

After 3 additional days infection in each test well was determined by observation of the cytopathic effect. Data were then used to determine the titer of treated and control virus according to Reed and Muench method (ref. Reed, L.J.; Muench, H. (1938). "A simple method of estimating fifty percent endpoints". The American Journal of Hygiene. 27: 493-49).

The results are depicted in figure 12.

**Antiviral effect (SARS-CoV-2 virus Delta strain) of a surface functionalized with Gly-DiaN-BA CDs.**

Surface functionalization

Silicon substrates were at first coated with a hybrid silica film bearing amino groups via sol-gel process, and then functionalized with Gly-Dian-BA through an EDC/NHS coupling reaction.

Experimental parameters:

A volume of viral suspension (5 μL) of against SARS-CoV-2 virus (Delta strain) was casted on a 96-well plate lid (12x8 cm). Then the viral suspension was added to the coated plates.

The substrates were then left in the dark or exposed to ultraviolet (UV = 365 nm) or visible light (VL = 450 nm) for 5 minutes. After an additional 5-minute incubation, viral suspensions were collected and viral titer was determined according to the methodology described below.

Viral titer determination

An in vitro system was employed to determine viral titer of SARS-CoV-2 suspensions.

Vero E6 cells (kidney epithelial cells from African green monkey, ATCC CRL-1586) were maintained at their optimal density based on ATCC data sheet. On Day 1 of the experiment cells were transferred in 96-well plate (20.000 cells per well). On Day 2 cells were infected with the serial viral dilutions (10-2, 10-3, 10-4...) in 6-well replicates for each condition. Importantly, treatment concentration of each nanomaterial was determined in order to get non-cytotoxic concentration at the first serial dilution added to cells.

After 3 additional days infection in each test well was determined by observation of the cytopathic effect. Data were then used to determine the titer of treated and control virus according to Reed and Muench method (ref. Reed, L.J.; Muench, H. (1938). "A simple method of estimating fifty percent endpoints". The American Journal of Hygiene. 27: 493-49).

The results are depicted in figure 13.

**Bactericidal effect of Gly-DiaN-BA CDs**

Slices of fruit were treated with slice treated with an aqueous solution of Gly-DiaN-BA Cdots according to the invention (concentration 1 mg/mL). The aqueous solution was sprayed on the strawberry slice at a distance of 20 cm and was to air at room temperature.

Untreated and treated slices from the same fruit (strawberry) were kept at room temperature under different conditions, changes in the slices were monitored over time. The results of the observation in time are reported in Figure 14. The figure shows untreated slices with extensive mould growth and rottening after 3 days (14a) whereas treated slices (14b) show signs of drying but no mould or rottening after 196 days.

Antifungal and a general antimicrobial effect of the CDs of the invention are evident from the results of the experiment.

## Claims

1. A process for the preparation of carbon dots comprising

    a. dissolving Glycine, 1,5-diaminonaphthalene and boric acid in distilled water,
    b. sonicating the mixture obtained in a. until the solution becomes turbid grey
    c. submitting the turbid grey solution thus obtained to thermal treatment and solubilising the product thereby obtained in distilled water
    d. filtering the mixture obtained in c. with a 10-30 μm pore size filter and collecting the eluate
    e. drying the eluate thus obtained

    wherein Glycine, 1,5-diaminonaphthalene, boric acid and water are the sole compounds used in said process.

2. The process of claim 1 wherein the ratio in weight of Glycine: 1,5-diaminonaphtalene:boric acid in a. is of about 1.0:2.0:0.8; preferably the ratio is of 1.0:2.06:0.8, or wherein 0.15 g of Glycine, 0.31 g of 1,5-diaminonaphthalene and 0.12 g of boric acid are dissolved in 15-25 ml of distilled water, preferably in 20 ml of distilled water.

3. The process of anyone of claims 1 to 2 wherein said thermal treatment is carried out for a time period of 2-5 minutes at 800-1200 W in a microwave oven or in an oven at 200°C for a time range not shorter than 12 h or in an autoclave under hydrothermal conditions at 200°C for at least 8 h and/or wherein said drying is carried out by submitting said eluate at a temperature of 50-70°C for a period of time of 12-18 hours.

4. Carbon dots **characterised in that** they have a maximum emission peak between 357nm and 425 nm in the 300-700 nm wavelength range under excitation at 320 nm and a maximum absorbance peak between 310 nm and 340 nm in the 200-600 nm wavelength range when measured at a carbon dots concentration of 0.1 mg/ml in water.

5. The carbon dots according to claim 4 wherein said carbon dots are obtainable with the process according to anyone of claims 1 to 3.

6. A composition comprising the carbon dots of anyone of claims 4 to 5 and at least one carrier and/or excipient, preferably wherein said composition is a pharmaceutical composition and said at least one carrier and/or excipient is pharmaceutically acceptable.

7. A product coated or loaded with the carbon dots of anyone of claims 4 to 5 or with the composition of claim 6.

8. The product of claim 7 wherein said product is a sanitizing product, a cleaning product, a medical device, a medical equipment, an individual protection device, a system for air filtration, a surgical mask, a filtering facepiece respirator, a surgical instrument, a device comprising a reservoir for the storage of said carbon dots or composition and means for the release of said carbon dots or composition.

9. A medical device comprising the carbon dots of anyone of claims 4 to 5 or the pharmaceutical composition of claim 6, preferably wherein said medical device is a drug delivery device, preferably wherein said device comprises means for the emission of visible or UV light or being transparent to visible and/or UV light.

10. The carbon dots of anyone of claims 4 to 5 or the pharmaceutical composition of claim 6 for use in a medical treatment.

**11.** The carbon dot or the pharmaceutical composition for use according to claim 10 for use in the treatment of a microbial infection or as adjuvant in a treatment of a microbial infection or in a treatment for the prevention of a microbial infection or of an infectious disease, preferably wherein said infection affects an animal or a plant.

**12.** The carbon dot or the pharmaceutical composition for use according to claim 10 or 11 wherein said microbial infection is a viral infection, a bacterial infection, a fungal infection, a phytoplasma infection, preferably wherein said infection is a viral infection caused by an animal DNA virus or an animal RNA virus, even more preferably wherein said animal is a mammalian.

**13.** The carbon dot or the pharmaceutical composition for use according to claim 12 wherein said infection is caused by a Coronavirus, an Orthomyxovirus, a Filovirus, a Flavivirus, an Hepadnavirus, a Hepevirus, a Herpesvirus, a Papillomavirus, a Pneumovirus, a Poxivirus, a Rhinovirus, a Reovirus, a Togavirus, an influenza virus, preferably wherein said Coronavirus is SARS, MERS or SARS-CoV-2, FCoV.

**14.** The use of the carbon dots of anyone of claims 4 to 5 of the composition of claim 6 for the disinfection, sanitisation and/or sterilisation of surfaces, objects, air, waters, areas.

**15.** A method for the disinfection, sanitisation and/or sterilisation of surfaces, objects, air, waters, areas wherein comprising
putting in contact the object of the disinfection, santitisation and/or sterilisation with a suitable amount of the carbon dots as defined in anyone of claims 4 to 5 of the composition as defined in claim 6 and subjecting it to irradiation with visible and/or UV light for a period of time of at least 5 minutes.

## Patentansprüche

**1.** Verfahren zur Herstellung von Kohlenstoffpunkten, umfassend

a. Lösen von Glycin, 1,5-Diaminonaphthalin und Borsäure in destilliertem Wasser,
b. Beschallen der unter a. erhaltenen Mischung, bis die Lösung trübgrau wird
c. Unterziehen der so erhaltenen trübgrauen Lösung einer thermischen Behandlung und Lösen des dabei erhaltenen Produkts in destilliertem Wasser
d. Filtrieren der in c. erhaltenen Mischung mit einem Filter mit einer Porengröße von 10-30 μm und Auffangen des Eluats
e. Trocknen des so erhaltenen Eluats,

wobei Glycin, 1,5-Diaminonaphthalin, Borsäure und Wasser die einzigen in diesem Verfahren verwendeten Verbindungen sind.

**2.** Verfahren nach Anspruch 1, wobei das Gewichtsverhältnis von Glycin : 1,5-Diaminonaphthalin : Borsäure in a. etwa 1,0 : 2,0 : 0,8 beträgt, vorzugsweise das Verhältnis 1,0 : 2,06 : 0,8 beträgt, oder wobei 0,15 g Glycin, 0,31 g 1,5-Diaminonaphthalin und 0,12 g Borsäure in 15-25 ml destilliertem Wasser, vorzugsweise in 20 ml destilliertem Wasser, gelöst werden.

**3.** Verfahren nach einem der Ansprüche 1 bis 2, wobei die Wärmebehandlung über einen Zeitraum von 2 bis 5 Minuten bei 800 bis 1200 W in einem Mikrowellenherd oder in einem Ofen bei 200 °C für einen Zeitraum von nicht weniger als 12 Stunden oder in einem Autoklaven unter hydrothermalen Bedingungen bei 200 °C für mindestens 8 Stunden durchgeführt wird und/oder wobei das Trocknen durchgeführt wird, indem das Eluat über einen Zeitraum von 12 bis 18 Stunden einer Temperatur von 50 bis 70 °C ausgesetzt wird.

**4.** Kohlenstoffpunkte, **dadurch gekennzeichnet, dass** sie einen maximalen Emissionspeak zwischen 357 nm und 425 nm im Wellenlängenbereich von 300-700 nm bei Anregung bei 320 nm und einen maximalen Absorptionspeak zwischen 310 nm und 340 nm im Wellenlängenbereich von 200-600 nm aufweisen, wenn sie bei einer Kohlenstoffpunktkonzentration von 0,1 mg/ml in Wasser gemessen werden.

**5.** Kohlenstoffpunkte nach Anspruch 4, wobei die Kohlenstoffpunkte mit dem Verfahren nach einem der Ansprüche 1 bis 3 erhältlich sind.

6. Zusammensetzung, die die Kohlenstoffpunkte nach einem der Ansprüche 4 bis 5 und mindestens einen Träger und/oder Hilfsstoff umfasst, wobei die Zusammensetzung vorzugsweise eine pharmazeutische Zusammensetzung ist und der mindestens eine Träger und/oder Hilfsstoff pharmazeutisch verträglich ist.

7. Produkt, das mit den Kohlenstoffpunkten nach einem der Ansprüche 4 bis 5 oder mit der Zusammensetzung nach Anspruch 6 beschichtet oder beladen ist.

8. Produkt nach Anspruch 7, wobei das Produkt ein Desinfektionsprodukt, ein Reinigungsprodukt, eine medizinische Vorrichtung, eine medizinische Ausrüstung, eine persönliche Schutzvorrichtung, ein System zur Luftfilterung, eine chirurgische Maske, eine filtrierende Atemschutzmaske, ein chirurgisches Instrument, eine Vorrichtung umfassend ein Reservoir zur Aufbewahrung der Kohlenstoffpunkte oder der Zusammensetzung und Mittel zur Freigabe der Kohlenstoffpunkte oder der Zusammensetzung ist.

9. Medizinische Vorrichtung, umfassend die Kohlenstoffpunkte nach einem der Ansprüche 4 bis 5 oder die pharmazeutische Zusammensetzung nach Anspruch 6, wobei es sich bei der medizinischen Vorrichtung vorzugsweise um eine Arzneimittelverabreichungsvorrichtung handelt, wobei die Vorrichtung vorzugsweise Mittel zur Emission von sichtbarem oder UV-Licht umfasst oder für sichtbares und/oder UV-Licht durchlässig ist.

10. Kohlenstoffpunkte nach einem der Ansprüche 4 bis 5 oder die pharmazeutische Zusammensetzung nach Anspruch 6 zur Verwendung bei einer medizinischen Behandlung.

11. Kohlenstoffpunkt oder die pharmazeutische Zusammensetzung zur Verwendung nach Anspruch 10 zur Verwendung bei der Behandlung einer mikrobiellen Infektion oder als Adjuvans bei der Behandlung einer mikrobiellen Infektion oder bei einer Behandlung zur Vorbeugung einer mikrobiellen Infektion oder einer Infektionskrankheit, vorzugsweise wobei die Infektion ein Tier oder eine Pflanze betrifft.

12. Kohlenstoffpunkt oder die pharmazeutische Zusammensetzung zur Verwendung nach Anspruch 10 oder 11, wobei die mikrobielle Infektion eine Virusinfektion, eine bakterielle Infektion, eine Pilzinfektion oder eine Phytoplasma-Infektion ist, wobei die Infektion vorzugsweise eine Virusinfektion ist, die durch ein tierisches DNA-Virus oder ein tierisches RNA-Virus verursacht wird, und wobei es sich bei dem Tier noch mehr bevorzugt um ein Säugetier handelt.

13. Kohlenstoffpunkt oder die pharmazeutische Zusammensetzung zur Verwendung nach Anspruch 12, wobei die Infektion durch ein Coronavirus, ein Orthomyxovirus, ein Filovirus, ein Flavivirus, ein Hepadnavirus, ein Hepevirus, ein Herpesvirus, ein Papillomavirus, ein Pneumovirus, ein Poxivirus, ein Rhinovirus, ein Reovirus, ein Togavirus oder ein Influenzavirus verursacht wird, wobei es sich bei dem Coronavirus vorzugsweise um SARS, MERS oder SARS-CoV-2, FCoV handelt.

14. Verwendung der Kohlenstoffpunkte nach einem der Ansprüche 4 bis 5 der Zusammensetzung nach Anspruch 6 zur Desinfektion, Sanitisierung und/oder Sterilisation von Oberflächen, Gegenständen, Luft, Gewässern, Bereichen.

15. Verfahren zur Desinfektion, Sanitisierung und/oder Sterilisation von Oberflächen, Gegenständen, Luft, Wasser, Bereichen, wobei es umfasst
Inkontaktbringen des zu desinfizierenden, zu sanitisierenden und/oder zu sterilisierenden Objekts mit einer geeigneten Menge der Kohlenstoffpunkte nach einem der Ansprüche 4 bis 5 der Zusammensetzung nach Anspruch 6 und Aussetzen desselben einer Bestrahlung mit sichtbarem und/oder UV-Licht für einen Zeitraum von mindestens 5 Minuten.

## Revendications

1. Procédé de préparation de points de carbone comprenant

a. la dissolution de glycine, de 1,5-diaminonaphtalène et d'acide borique dans de l'eau distillée,
b. la sonication du mélange obtenu en a. jusqu'à ce que la solution devienne grise trouble
c. la soumission de la solution grise trouble ainsi obtenue à un traitement thermique et la solubilisation du produit ainsi obtenu dans de l'eau distillée
d. la filtration du mélange obtenu en c. avec un filtre dont les pores ont une taille de 10-30 $\mu$m et la collecte de l'éluat

e. le séchage de l'éluat ainsi obtenu

dans lequel la glycine, le 1,5-diaminonaphtalène, l'acide borique et l'eau sont les seuls composés utilisés dans ledit procédé.

**2.** Procédé selon la revendication 1 dans lequel le rapport en poids de la glycine : du 1,5-diaminonaphtalène : de l'acide borique en a. est d'environ 1,0:2,0:0,8 ; de préférence, le rapport est de 1,0:2,06:0,8, ou dans lequel 0,15 g de glycine, 0,31 g de 1,5-diaminonaphtalène et 0,12 g d'acide borique sont dissous dans 15-25 ml d'eau distillée, de préférence dans 20 ml d'eau distillée.

**3.** Procédé selon l'une quelconque des revendications 1 à 2 dans lequel ledit traitement thermique est effectué pendant un laps de temps de 2-5 minutes à 800-1 200 W dans un four à micro-ondes ou dans un four à 200 °C pendant une durée non inférieure à 12 h ou dans un autoclave dans des conditions hydrothermales à 200 °C pendant au moins 8 h et/ou dans lequel ledit séchage est effectué en soumettant ledit éluat à une température de 50-70 °C pendant un laps de temps de 12-18 heures.

**4.** Points de carbone **caractérisés en ce qu'** ils ont un pic d'émission maximal entre 357 nm et 425 nm dans la gamme de longueurs d'onde 300-700 nm sous excitation à 320 nm et un pic d'absorbance maximal entre 310 nm et 340 nm dans la gamme de longueurs d'onde 200-600 nm lorsqu'ils sont mesurés à une concentration de points de carbone de 0,1 mg/ml dans l'eau.

**5.** Points de carbone selon la revendication 4, dans lesquels lesdits points de carbone peuvent être obtenus par le procédé selon l'une quelconque des revendications 1 à 3.

**6.** Composition comprenant les points de carbone selon l'une quelconque des revendications 4 à 5 et au moins un support et/ou un excipient, de préférence dans laquelle ladite composition est une composition pharmaceutique et ledit au moins un support et/ou excipient est pharmaceutiquement acceptable.

**7.** Produit enduit ou chargé avec les points de carbone selon l'une quelconque des revendications 4 à 5 ou avec la composition selon la revendication 6.

**8.** Produit selon la revendication 7, dans lequel ledit produit est un produit d'assainissement, un produit de nettoyage, un dispositif médical, un équipement médical, un dispositif de protection individuelle, un système de filtration de l'air, un masque chirurgical, un masque de protection respiratoire, un instrument chirurgical, un dispositif comprenant un réservoir pour le stockage desdits points de carbone ou de ladite composition et des moyens pour la libération desdits points de carbone ou de ladite composition.

**9.** Dispositif médical comprenant les points de carbone selon l'une quelconque des revendications 4 à 5 ou la composition pharmaceutique selon la revendication 6, de préférence dans lequel ledit dispositif médical est un dispositif d'administration de médicaments, de préférence dans lequel ledit dispositif comprend des moyens d'émission de lumière visible ou UV ou étant transparent à la lumière visible et/ou UV.

**10.** Points de carbone selon l'une quelconque des revendications 4 à 5 ou la composition pharmaceutique selon la revendication 6 pour une utilisation dans un traitement médical.

**11.** Point de carbone ou composition pharmaceutique pour une utilisation selon la revendication 10 pour une utilisation dans le traitement d'une infection microbienne ou comme adjuvant dans un traitement d'une infection microbienne ou dans un traitement pour la prévention d'une infection microbienne ou d'une maladie infectieuse, de préférence dans lequel ladite infection affecte un animal ou une plante.

**12.** Point de carbone ou composition pharmaceutique pour une utilisation selon la revendication 10 ou 11, dans lequel ladite infection microbienne est une infection virale, une infection bactérienne, une infection fongique, une infection à phytoplasme, de préférence dans lequel ladite infection est une infection virale causée par un virus à ADN animal ou un virus à ARN animal, encore plus préférablement dans lequel ledit animal est un mammifère.

**13.** Point de carbone ou composition pharmaceutique pour une utilisation selon la revendication 12, dans lequel ladite infection est provoquée par un Coronavirus, un Orthomyxovirus, un Filovirus, un Flavivirus, un Hepadnavirus, un Hepevirus, un Herpesvirus, un Papillomavirus, un Pneumovirus, un Poxivirus, un Rhinovirus, un Reovirus, un

Togavirus, un virus de la grippe, de préférence dans lequel ledit Coronavirus est le SARS, le MERS ou le SARS-CoV-2, le FCoV.

**14.** Utilisation des points de carbone selon l'une quelconque des revendications 4 à 5 ou de la composition selon la revendication 6 pour la désinfection, l'assainissement et/ou la stérilisation de surfaces, d'objets, de l'air, d'eaux, de zones.

**15.** Procédé de désinfection, d'assainissement et/ou de stérilisation de surfaces, d'objets, de l'air, d'eaux, de zones, comprenant
la mise en contact de l'objet de la désinfection, de l'assainissement et/ou de la stérilisation avec une quantité appropriée de points de carbone tels que définis dans l'une quelconque des revendications 4 à 5 de la composition telle que définie dans la revendication 6 et la soumission de cet objet à une irradiation par la lumière visible et/ou UV pendant un laps de temps d'au moins 5 minutes.

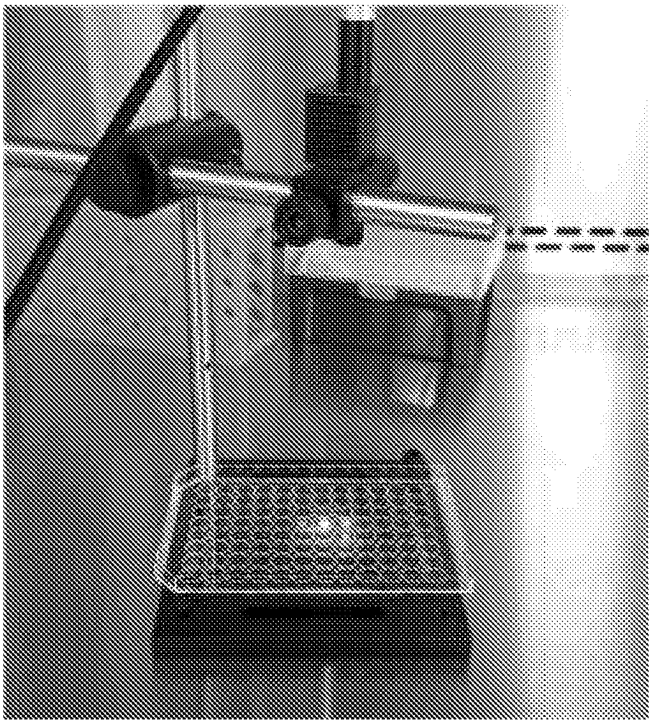

Fig. 1

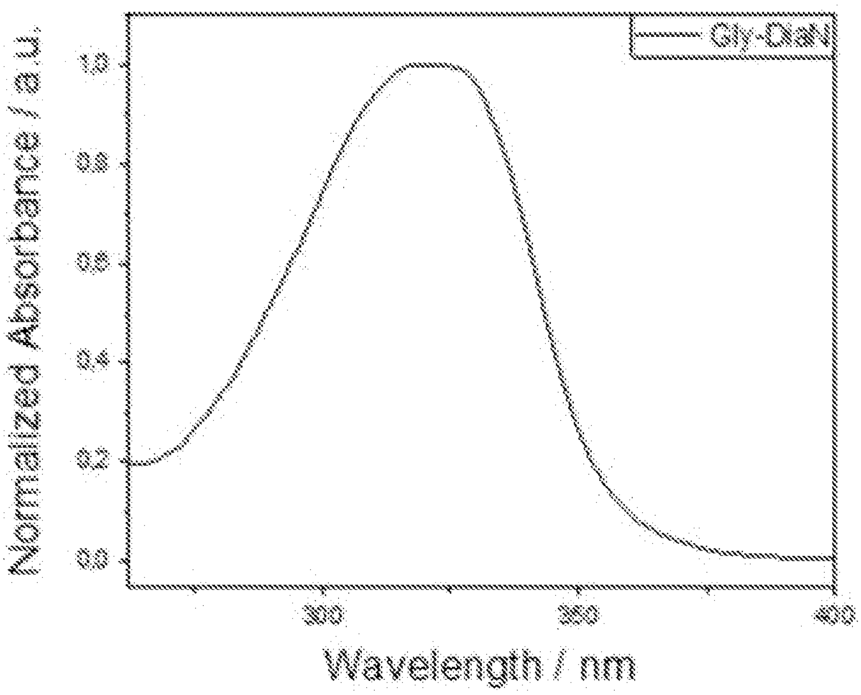
Gly-DiaN
Normalized Absorbance / a.u.
0.0
0.2
0.4
0.6
0.8
1.0
300
350
400
Wavelength / nm

Fig. 2A

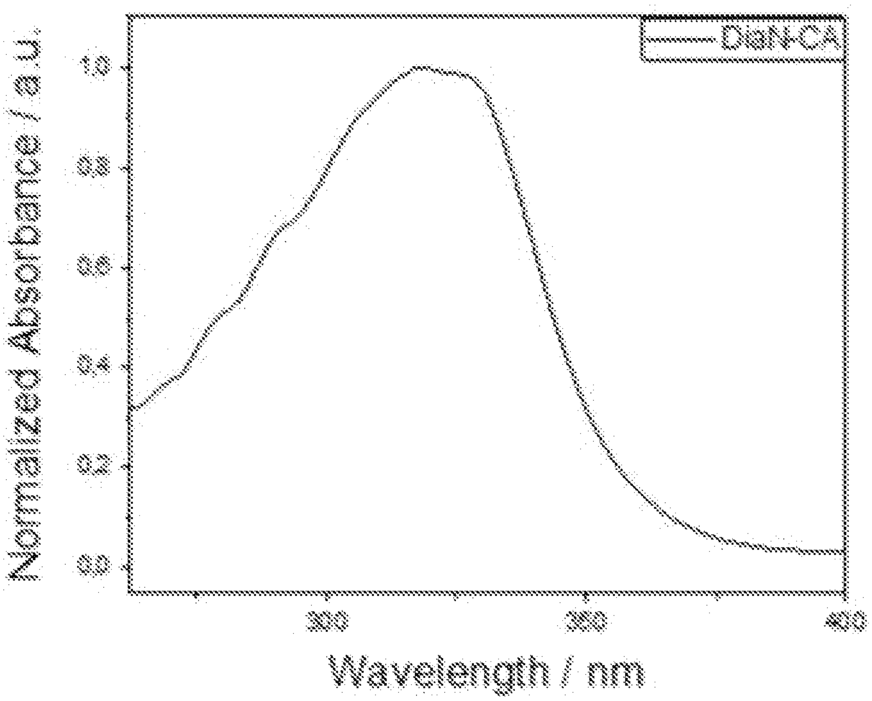
DiaN-CA
Normalized Absorbance / a.u.
1.0
0.8
0.6
0.4
0.2
0.0
300
350
400
Wavelength / nm

Fig. 2B

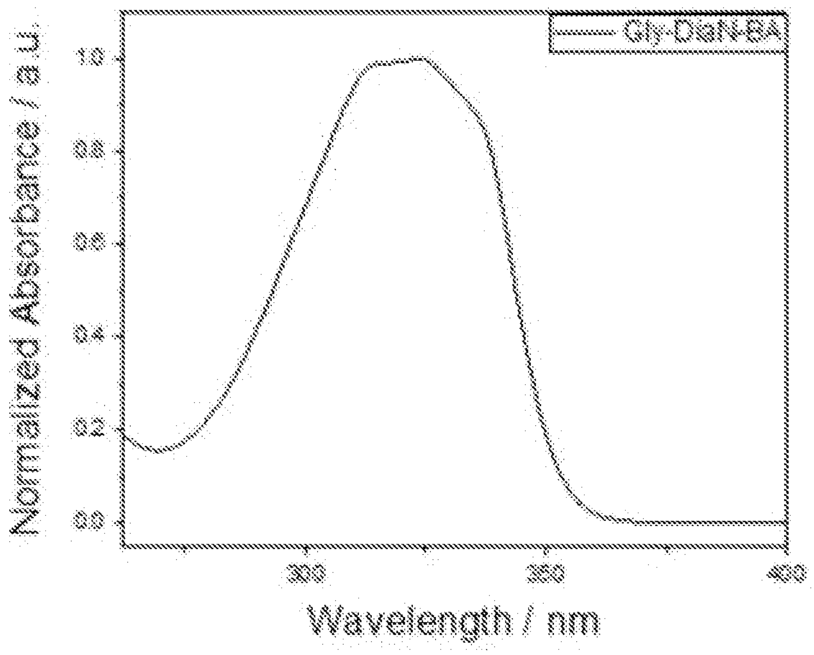
Gly-DiaN-BA
Normalized Absorbance / a.u.
1.0
0.8
0.6
0.4
0.2
0.0
300
350
400
Wavelength / nm

Fig. 2C

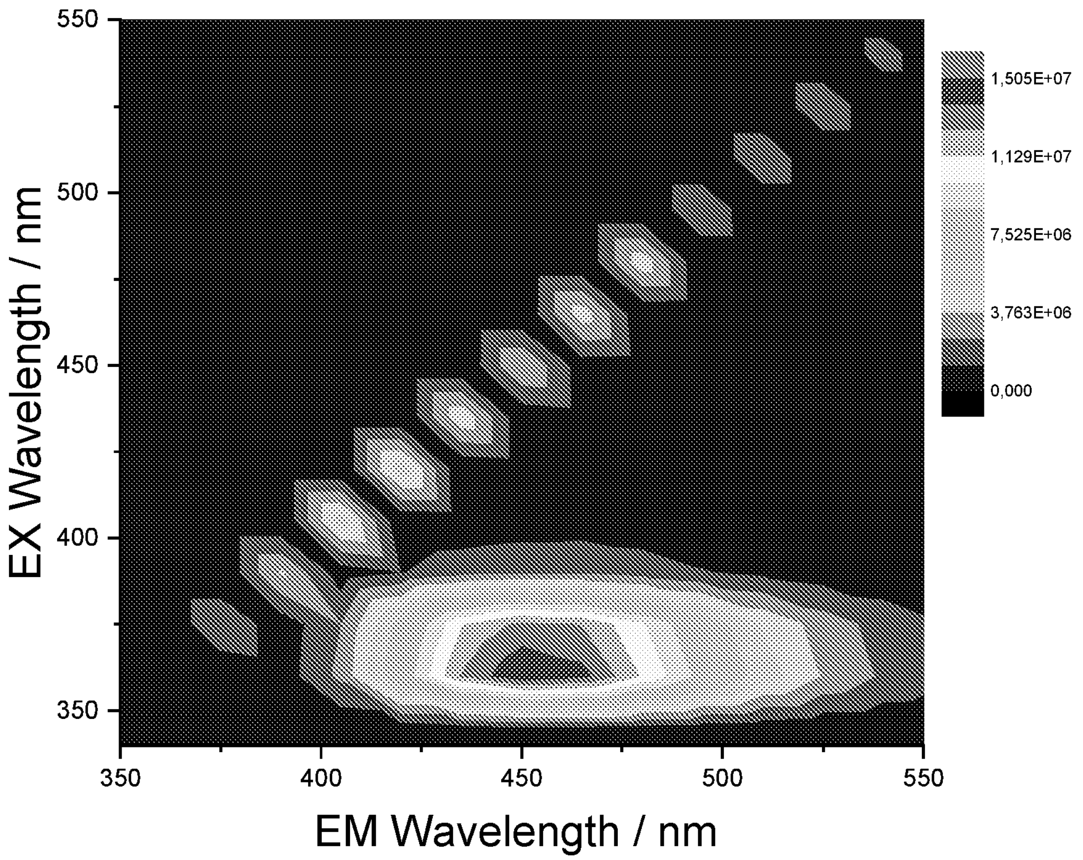
EX Wavelength / nm
550
500
450
400
350
EM Wavelength / nm
350
400
450
500
550
1,505E+07
1,129E+07
7,525E+06
3,763E+06
0,000

Fig. 3A

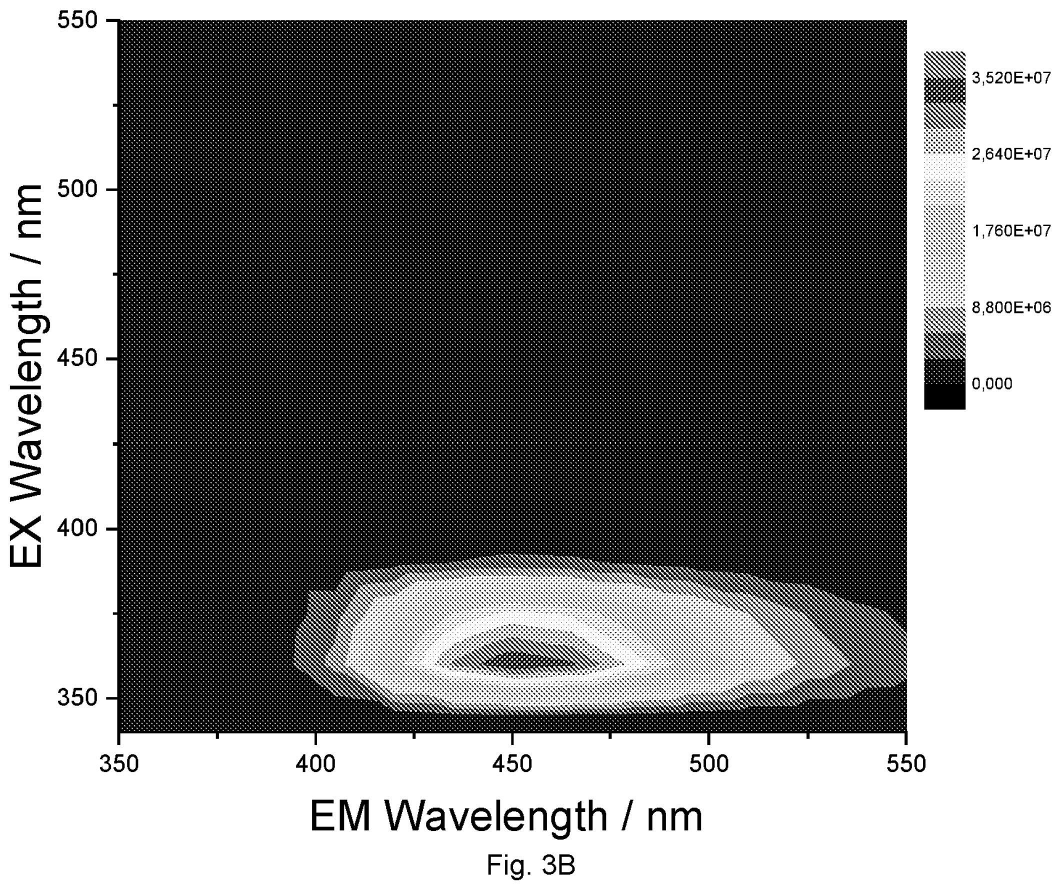
EX Wavelength / nm
550
500
450
400
350
EM Wavelength / nm
350
400
450
500
550
3,520E+07
2,640E+07
1,760E+07
8,800E+06
0,000

Fig. 3B

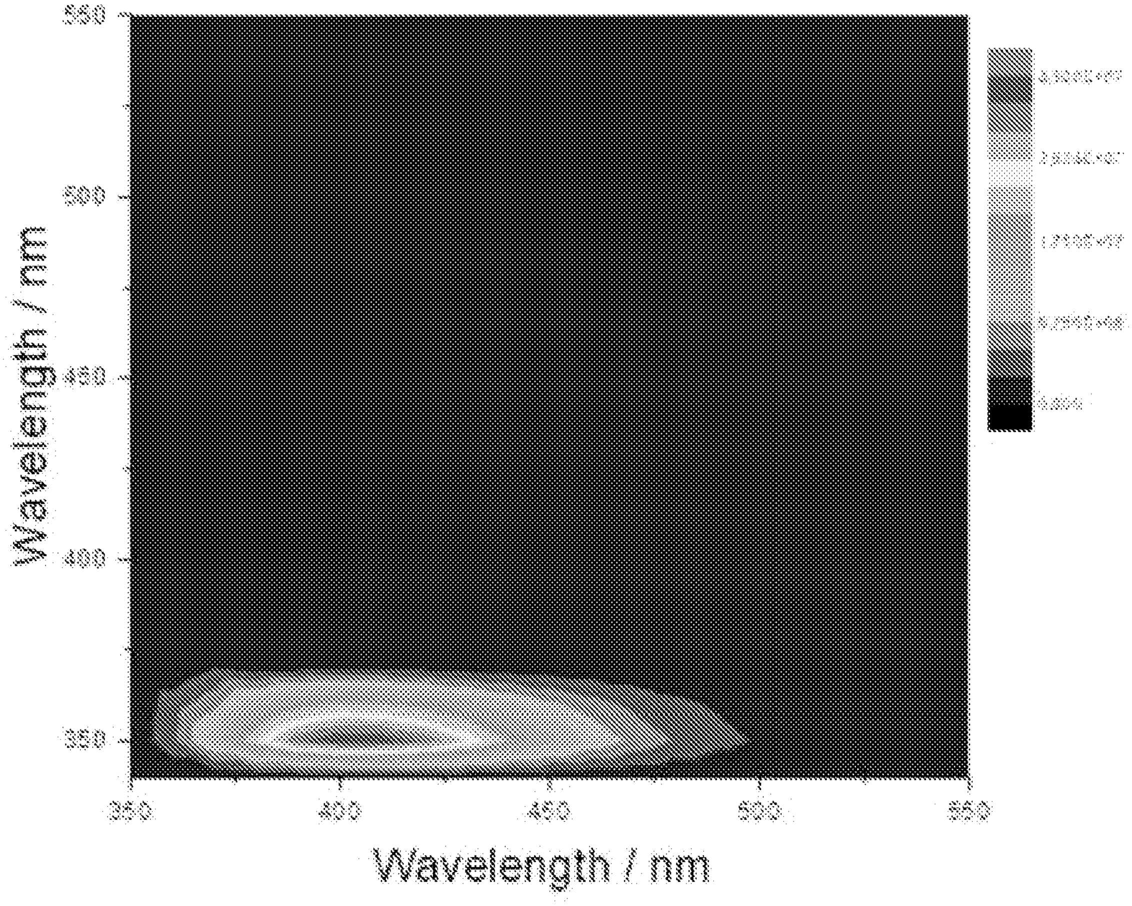
Wavelength / nm
Wavelength / nm
550
500
450
400
350
350
400
450
500
550

Fig. 3C

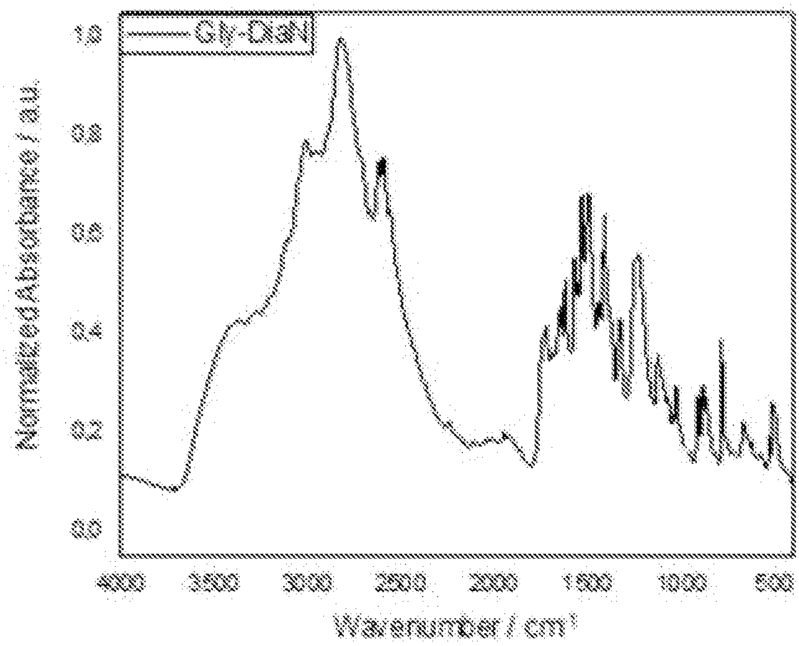
Gly-DiaN
Normalized Absorbance / a.u.
Wavenumber / cm-1
1.0
0.8
0.6
0.4
0.2
0.0
4000
3500
3000
2500
2000
1500
1000
500

Fig. 4A

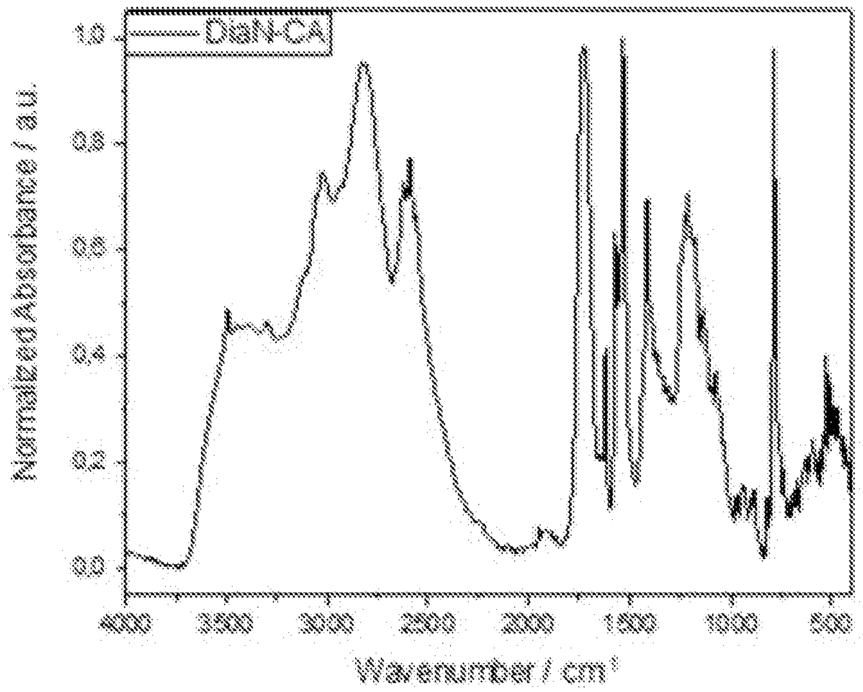
DiaN-CA
Normalized Absorbance / a.u.
Wavenumber / cm⁻¹
1.0
0.8
0.6
0.4
0.2
0.0
4000
3500
3000
2500
2000
1500
1000
500

Fig. 4B

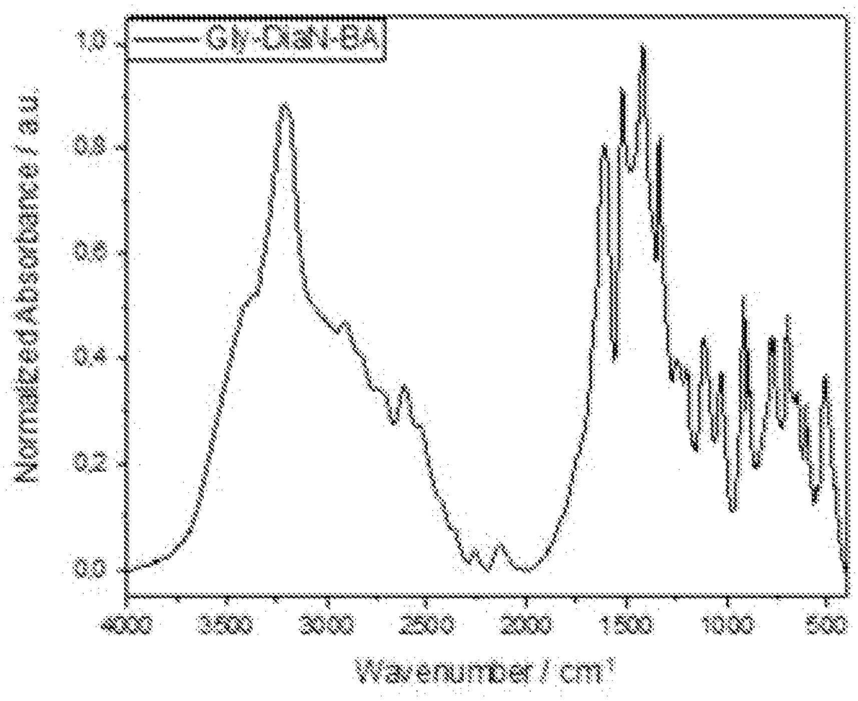
Gly-DiaN-BA
Normalized Absorbance / a.u.
Wavenumber / cm⁻¹
1.0
0.8
0.6
0.4
0.2
0.0
4000
3500
3000
2500
2000
1500
1000
500

Fig. 4C

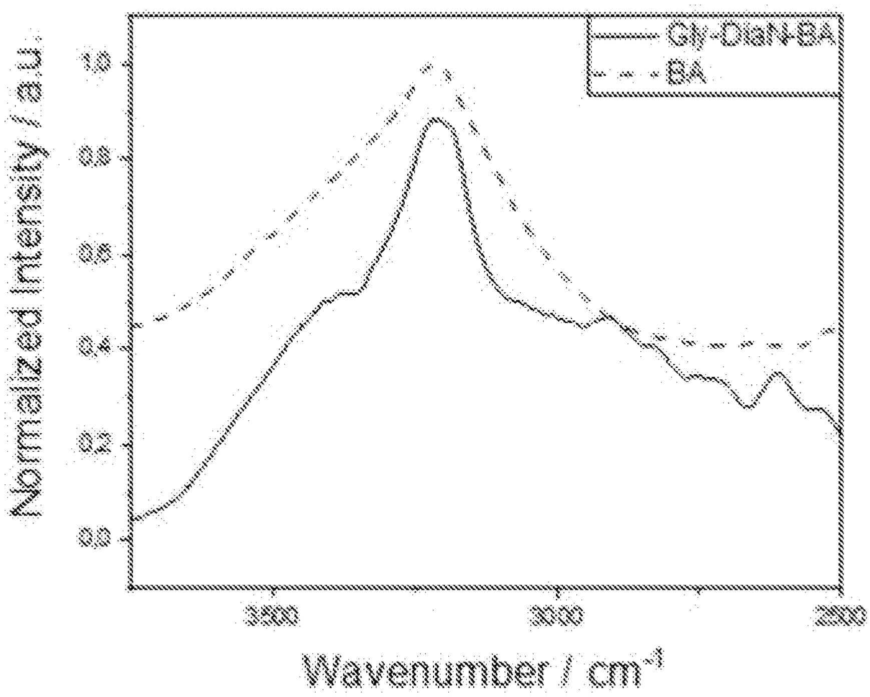
Gly-DiaN-BA
BA
Normalized Intensity / a.u.
1.0
0.8
0.6
0.4
0.2
0.0
3500
3000
2500
Wavenumber / cm-1

Fig. 4D

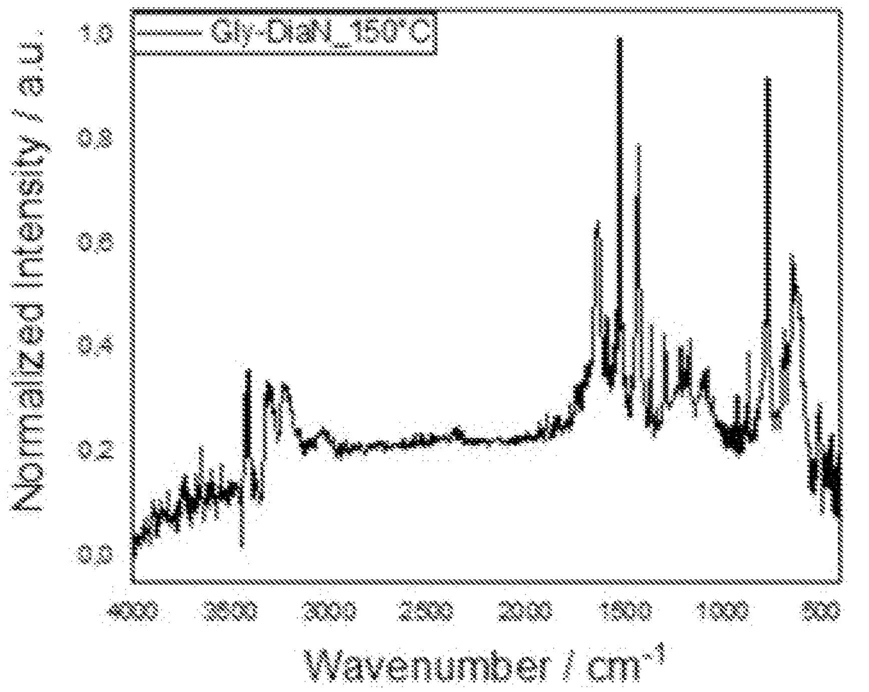
Gly-DiaN_150°C
Normalized Intensity / a.u.
1.0
0.8
0.6
0.4
0.2
0.0
4000
3500
3000
2500
2000
1500
1000
500
Wavenumber / cm-1

Fig. 5A

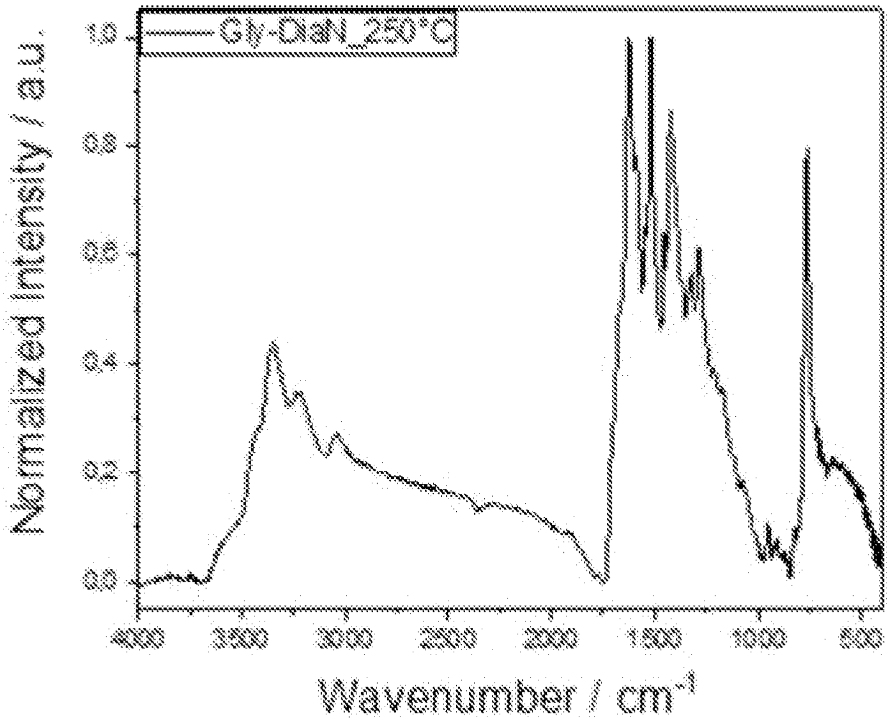
Gly-DiaN_250°C
Normalized Intensity / a.u.
Wavenumber / cm-1
1,0
0,8
0,6
0,4
0,2
0,0
4000
3500
3000
2500
2000
1500
1000
500

Fig. 5B

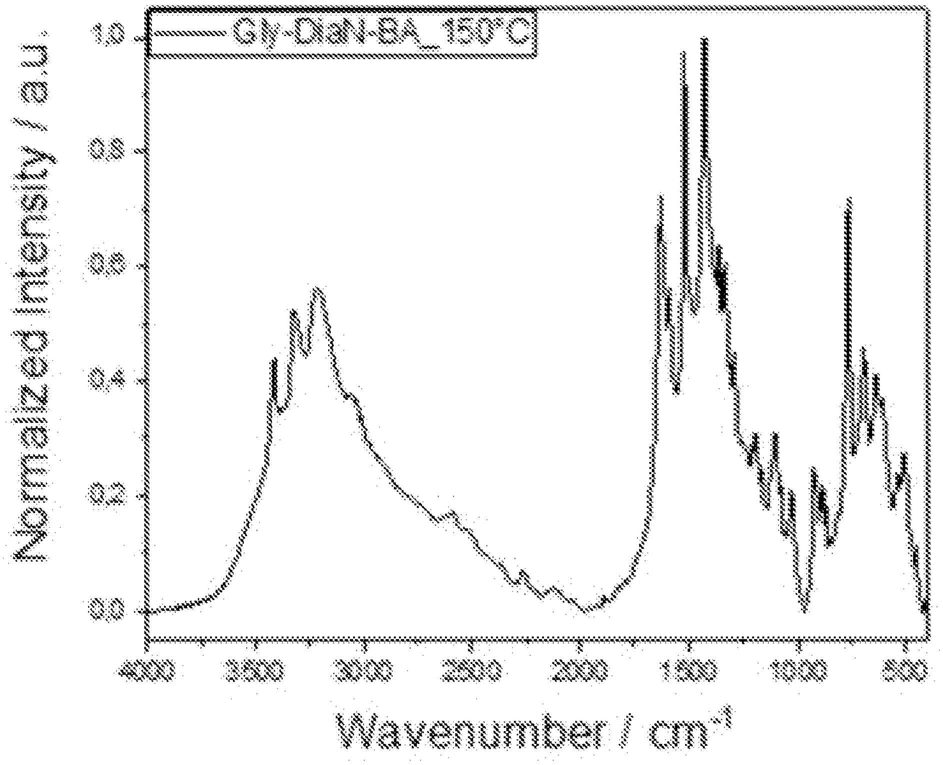
Gly-DiaN-BA_150°C
Normalized Intensity / a.u.
Wavenumber / cm-1
1,0
0,8
0,6
0,4
0,2
0,0
4000
3500
3000
2500
2000
1500
1000
500

Fig. 5C

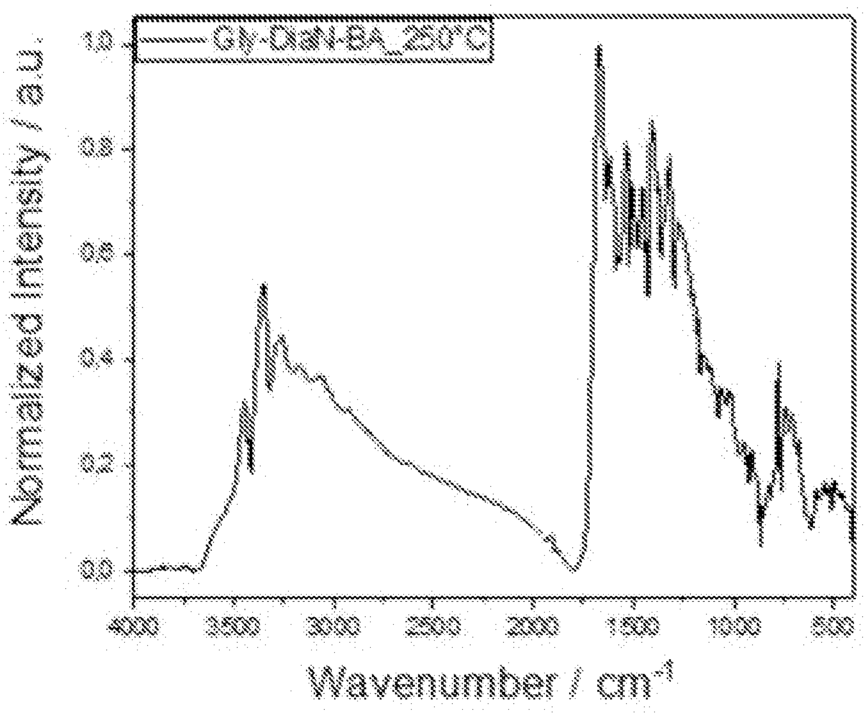
Gly-DiaN-BA_250°C
Normalized Intensity / a.u.
1.0
0.8
0.6
0.4
0.2
0.0
4000
3500
3000
2500
2000
1500
1000
500
Wavenumber / cm-1

Fig. 5D

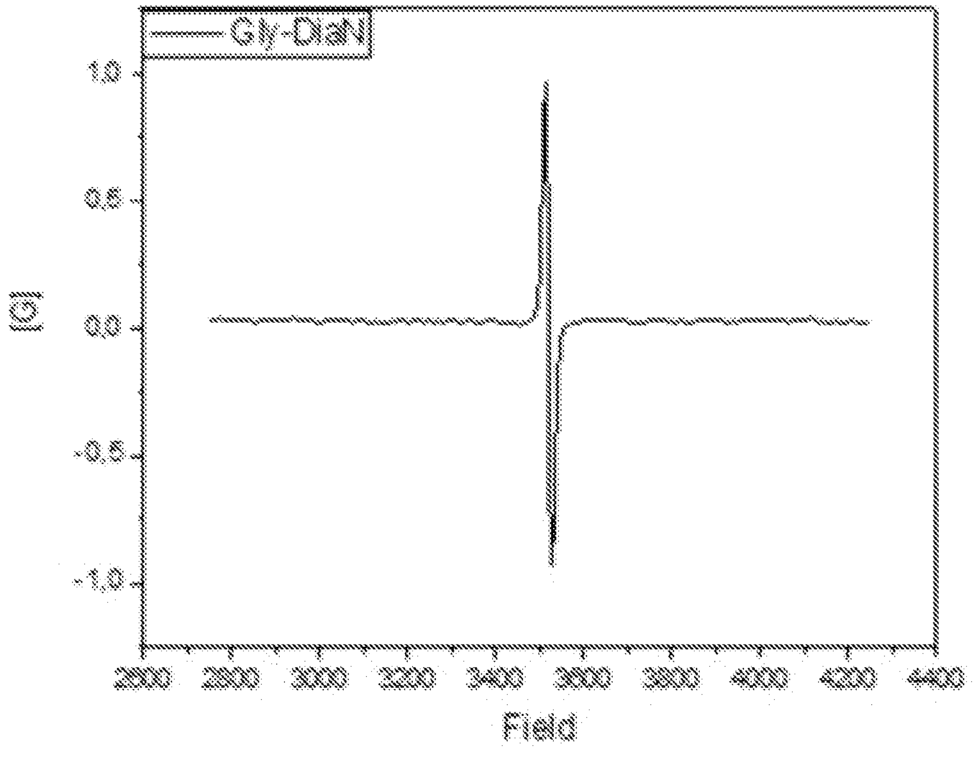
Gly-DiaN
[G]
1.0
0.5
0.0
-0.5
-1.0
2600
2800
3000
3200
3400
3600
3800
4000
4200
4400
Field

Fig. 6A

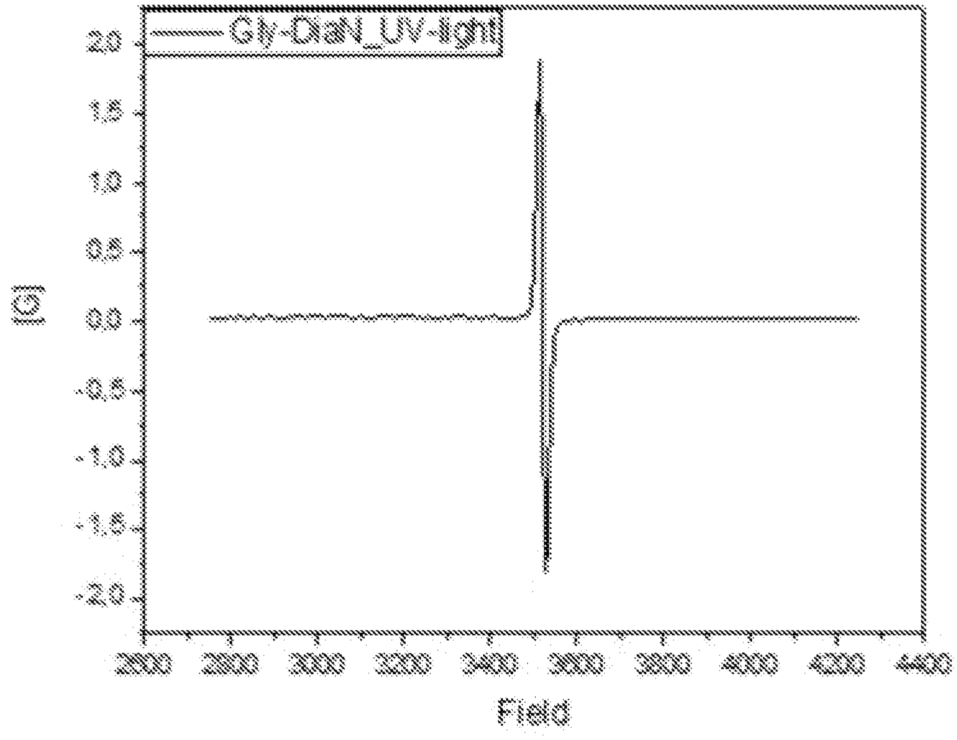
Gly-DiaN_UV-light
2,0
1,5
1,0
0,5
0,0
-0,5
-1,0
-1,5
-2,0
[G]
2600
2800
3000
3200
3400
3600
3800
4000
4200
4400
Field

Fig. 6B

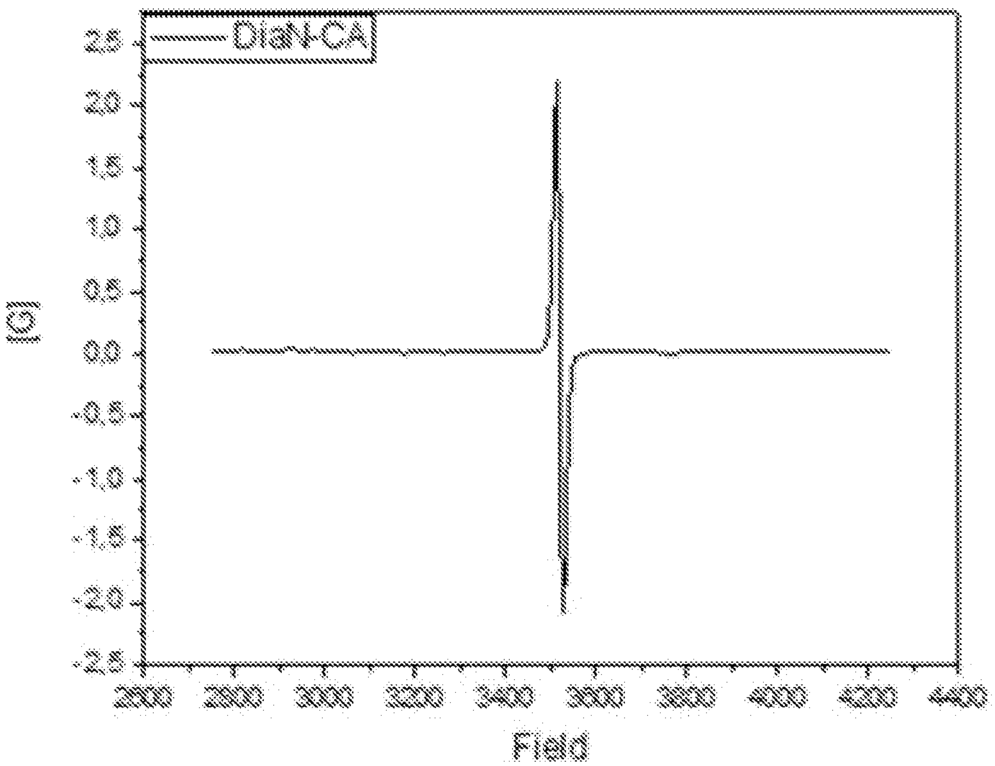
DiaN-CA
2,5
2,0
1,5
1,0
0,5
0,0
-0,5
-1,0
-1,5
-2,0
-2,5
[G]
2600
2800
3000
3200
3400
3600
3800
4000
4200
4400
Field

Fig. 6C

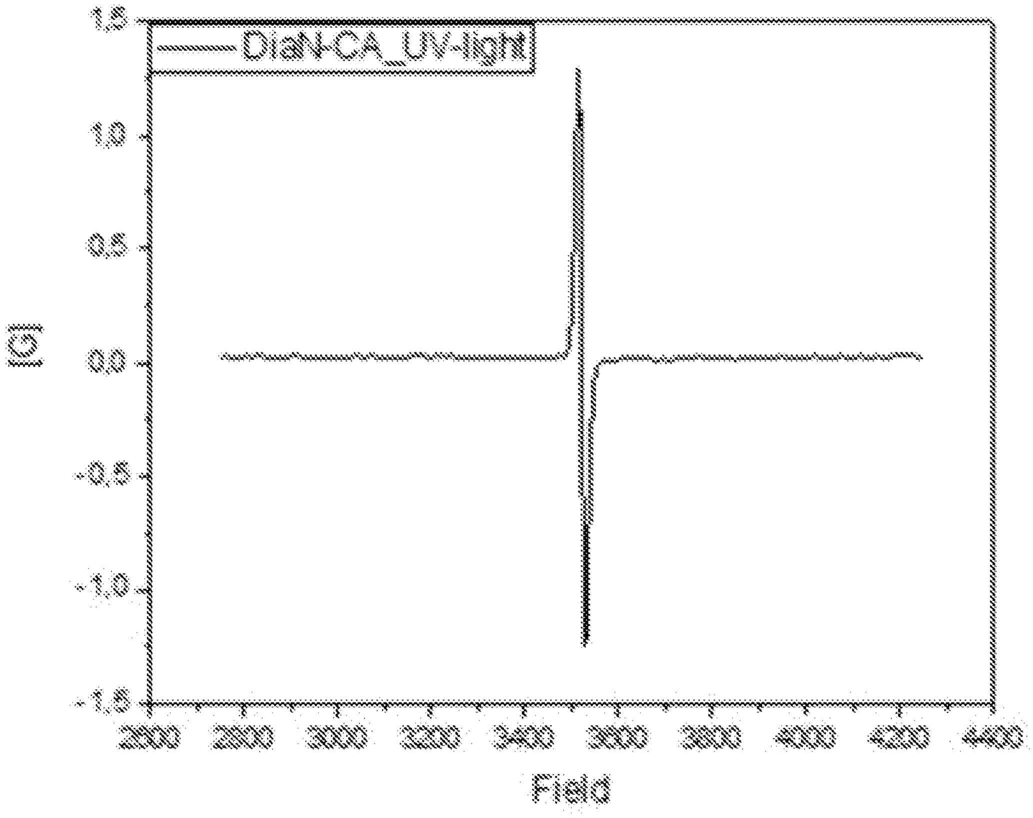
DiaN-CA_UV-light
1,5
1,0
0,5
0,0
-0,5
-1,0
-1,5
[G]
2600
2800
3000
3200
3400
3600
3800
4000
4200
4400
Field

Fig. 6D

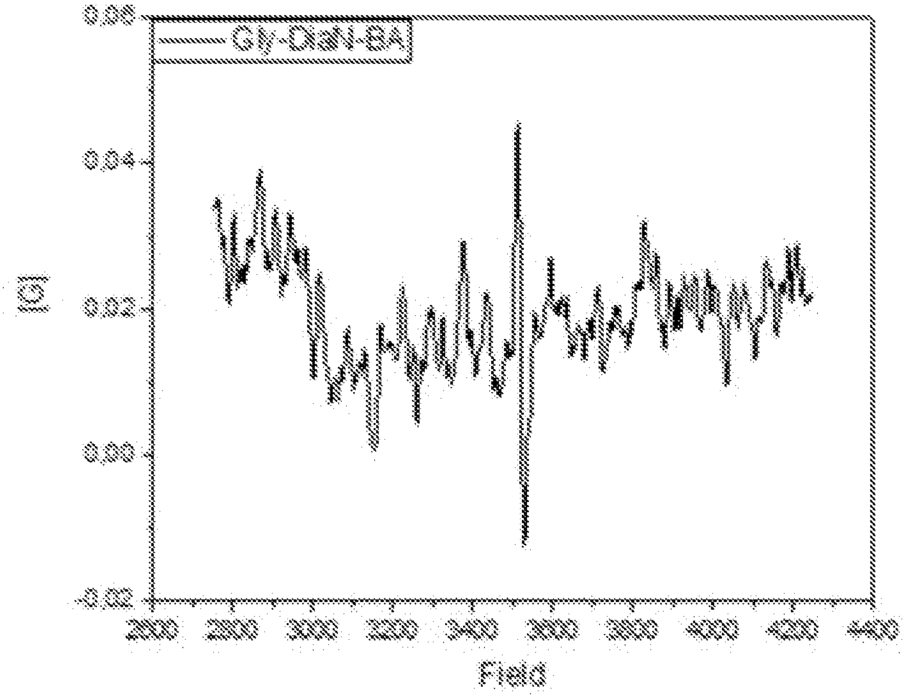
Gly-DiaN-BA
0,06
0,04
0,02
0,00
-0,02
[G]
2600
2800
3000
3200
3400
3600
3800
4000
4200
4400
Field

Fig. 6E

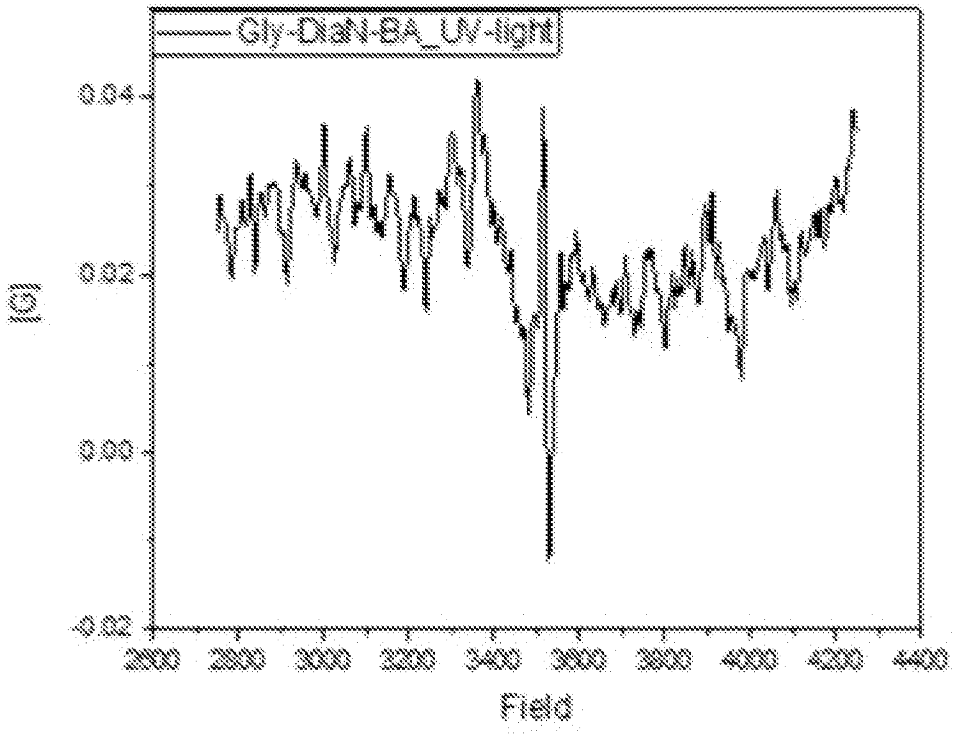
Gly-DiaN-BA_UV-light
0.04
0.02
0.00
-0.02
[G]
2600
2800
3000
3200
3400
3600
3800
4000
4200
4400
Field

Fig. 6F

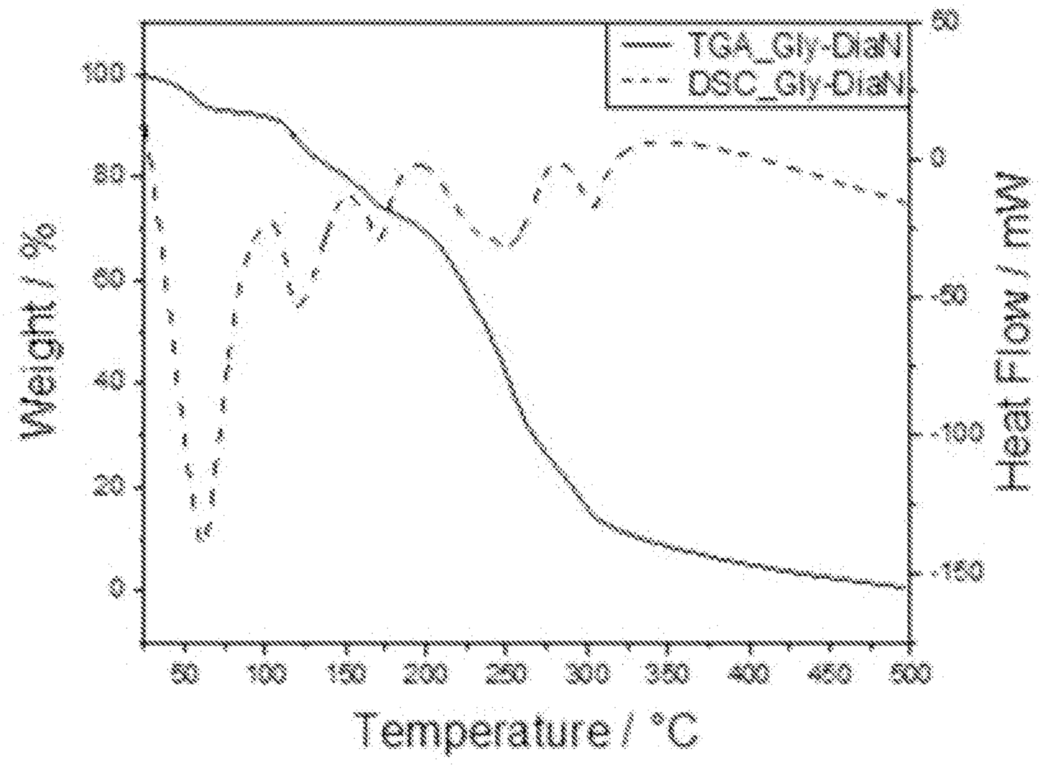
TGA_Gly-DiaN
DSC_Gly-DiaN
Weight / %
Heat Flow / mW
100
80
60
40
20
0
50
0
-50
-100
-150
50
100
150
200
250
300
350
400
450
500
Temperature / °C

Fig. 7A

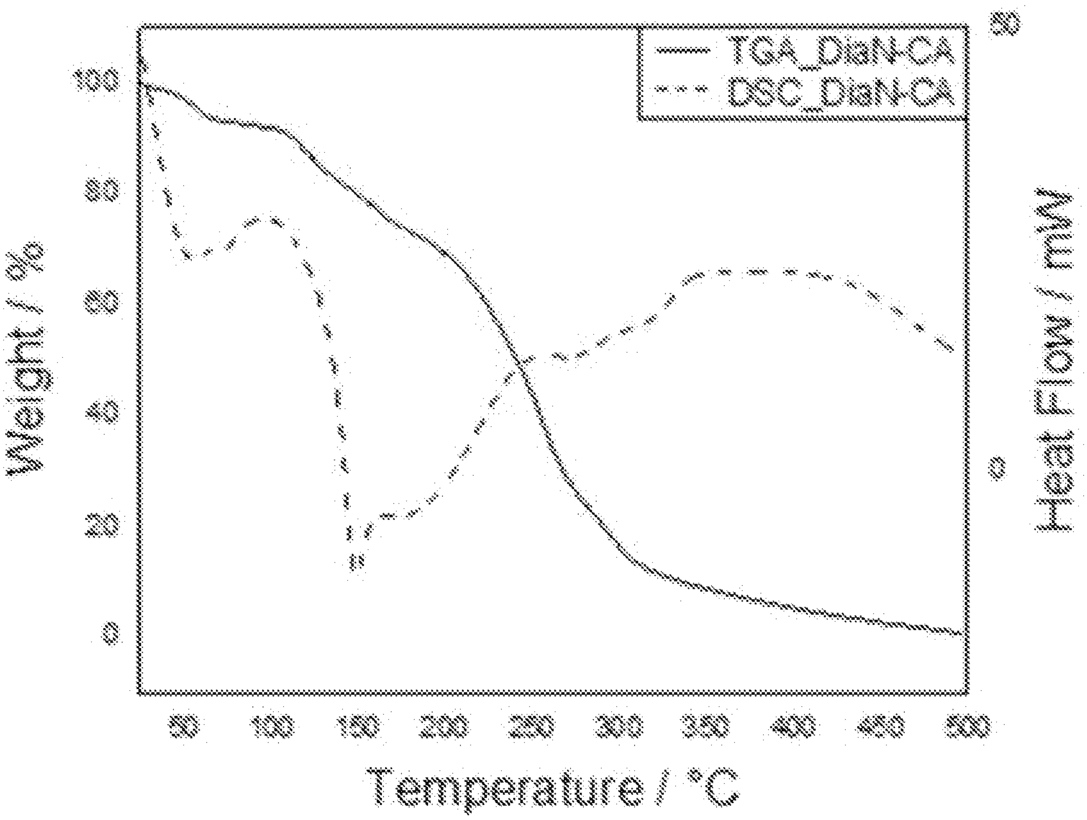
TGA_DiaN-CA
DSC_DiaN-CA
Weight / %
Heat Flow / mW
Temperature / °C
100
80
60
40
20
0
50
0
50
100
150
200
250
300
350
400
450
500

Fig. 7B

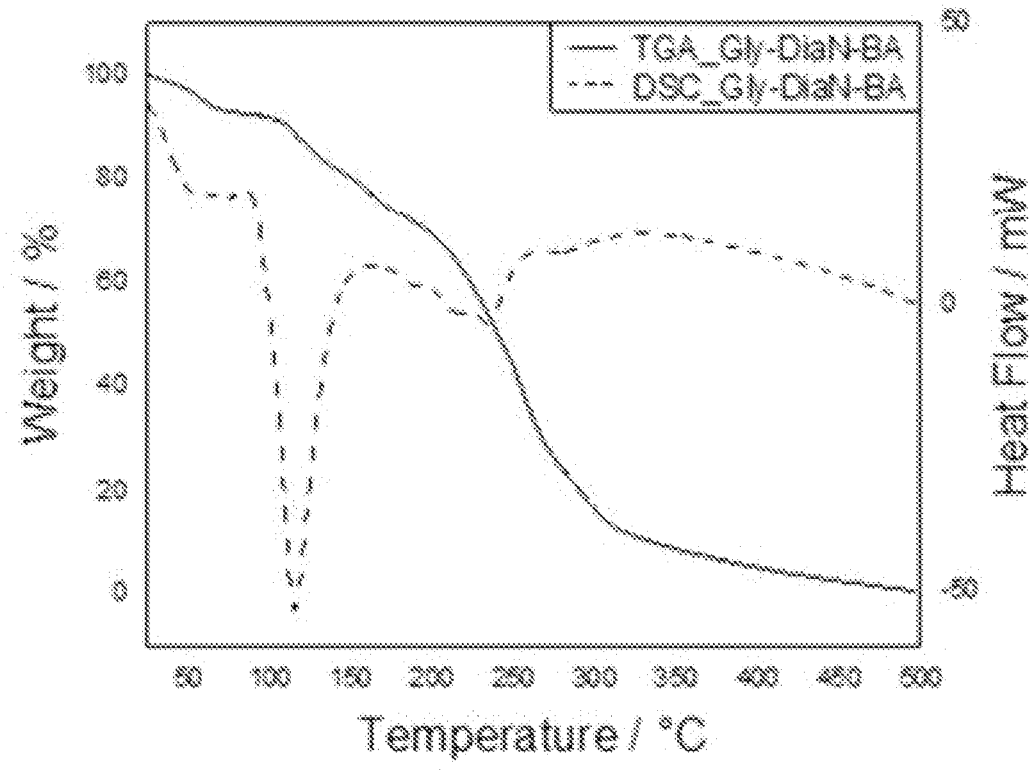
TGA_Gly-DiaN-BA
DSC_Gly-DiaN-BA
Weight / %
Heat Flow / mW
Temperature / °C
100
80
60
40
20
0
50
0
-50
50
100
150
200
250
300
350
400
450
500

Fig. 7C

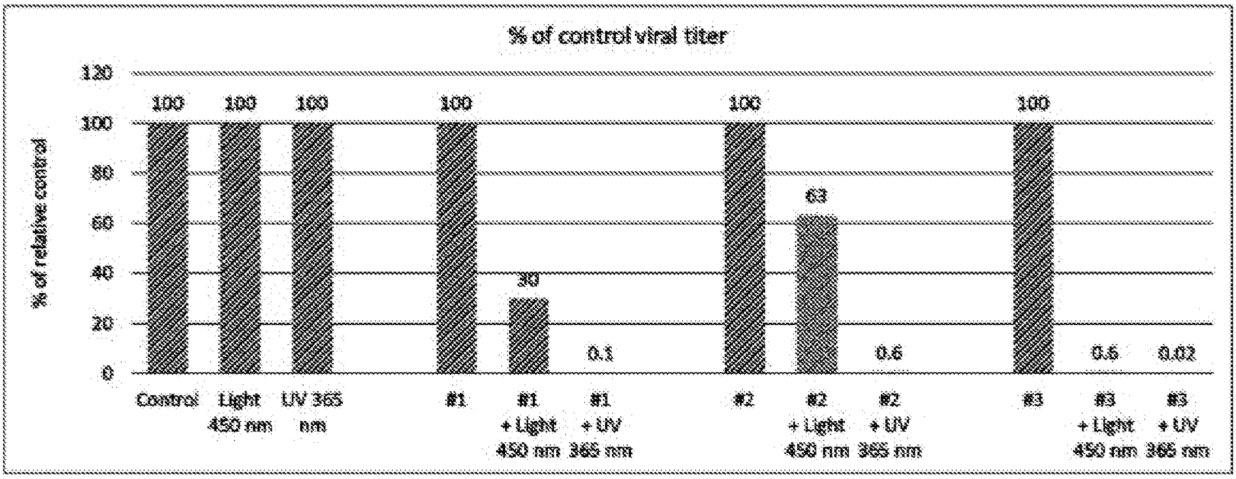
% of control viral titer
% of relative control
120
100
80
60
40
20
0
100
100
100
100
30
0.1
100
63
0.6
100
0.6
0.02
Control
Light 450 nm
UV 365 nm
#1
#1 + Light 450 nm
#1 + UV 365 nm
#2
#2 + Light 450 nm
#2 + UV 365 nm
#3
#3 + Light 450 nm
#3 + UV 365 nm

Fig. 8

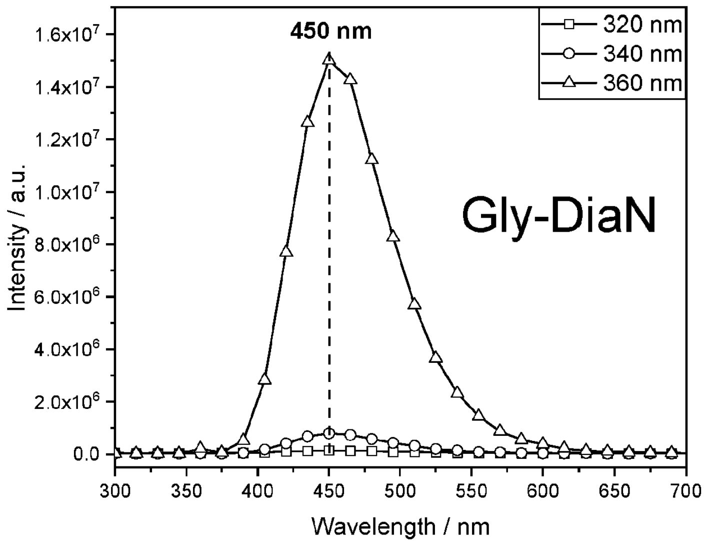
450 nm
320 nm
340 nm
360 nm
Gly-DiaN
Intensity / a.u.
Wavelength / nm
1.6x10^7
1.4x10^7
1.2x10^7
1.0x10^7
8.0x10^6
6.0x10^6
4.0x10^6
2.0x10^6
0.0
300
350
400
450
500
550
600
650
700

Fig. 9a

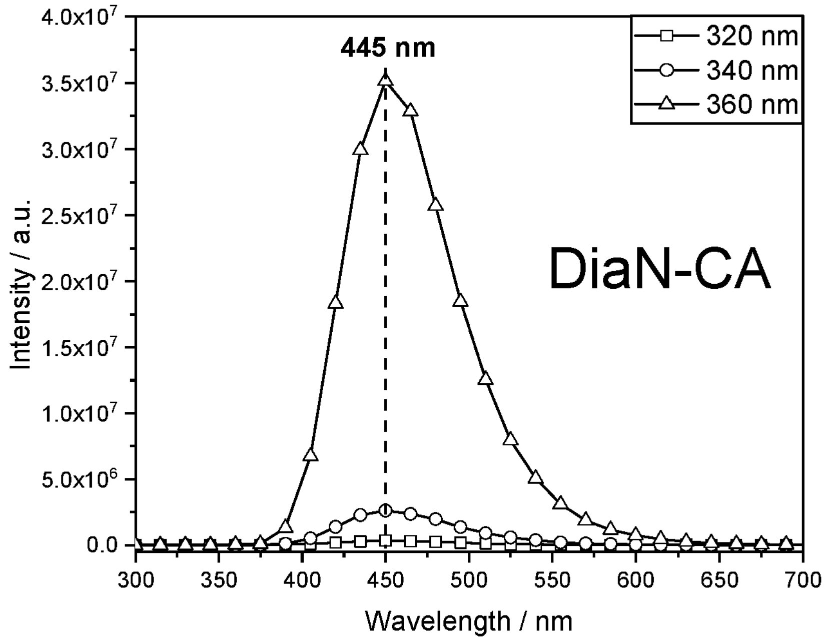
445 nm
320 nm
340 nm
360 nm
DiaN-CA
Intensity / a.u.
Wavelength / nm
4.0x10^7
3.5x10^7
3.0x10^7
2.5x10^7
2.0x10^7
1.5x10^7
1.0x10^7
5.0x10^6
0.0
300
350
400
450
500
550
600
650
700

Fig. 9b

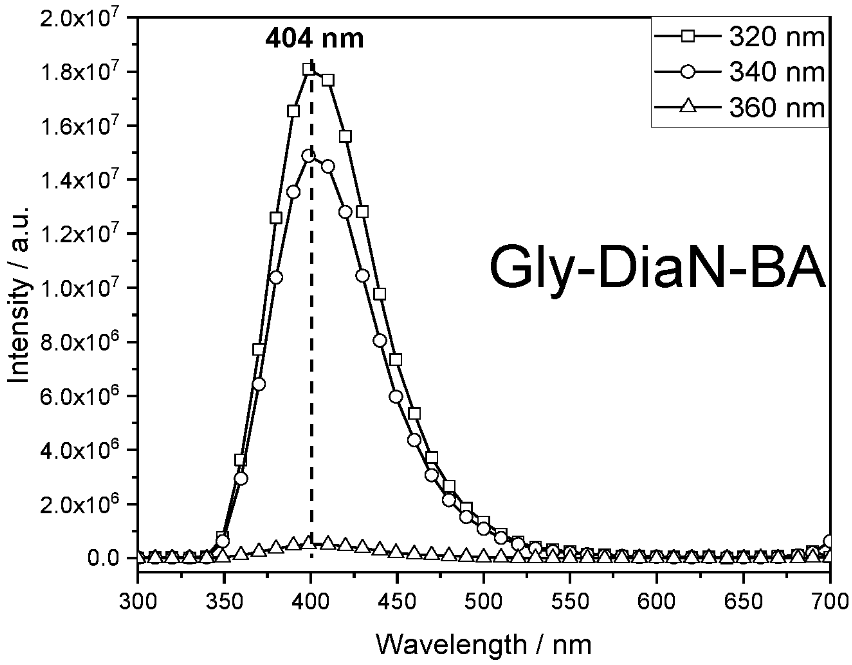
404 nm
320 nm
340 nm
360 nm
Gly-DiaN-BA
Intensity / a.u.
Wavelength / nm
2.0x10^7
1.8x10^7
1.6x10^7
1.4x10^7
1.2x10^7
1.0x10^7
8.0x10^6
6.0x10^6
4.0x10^6
2.0x10^6
0.0
300
350
400
450
500
550
600
650
700

Fig. 9c

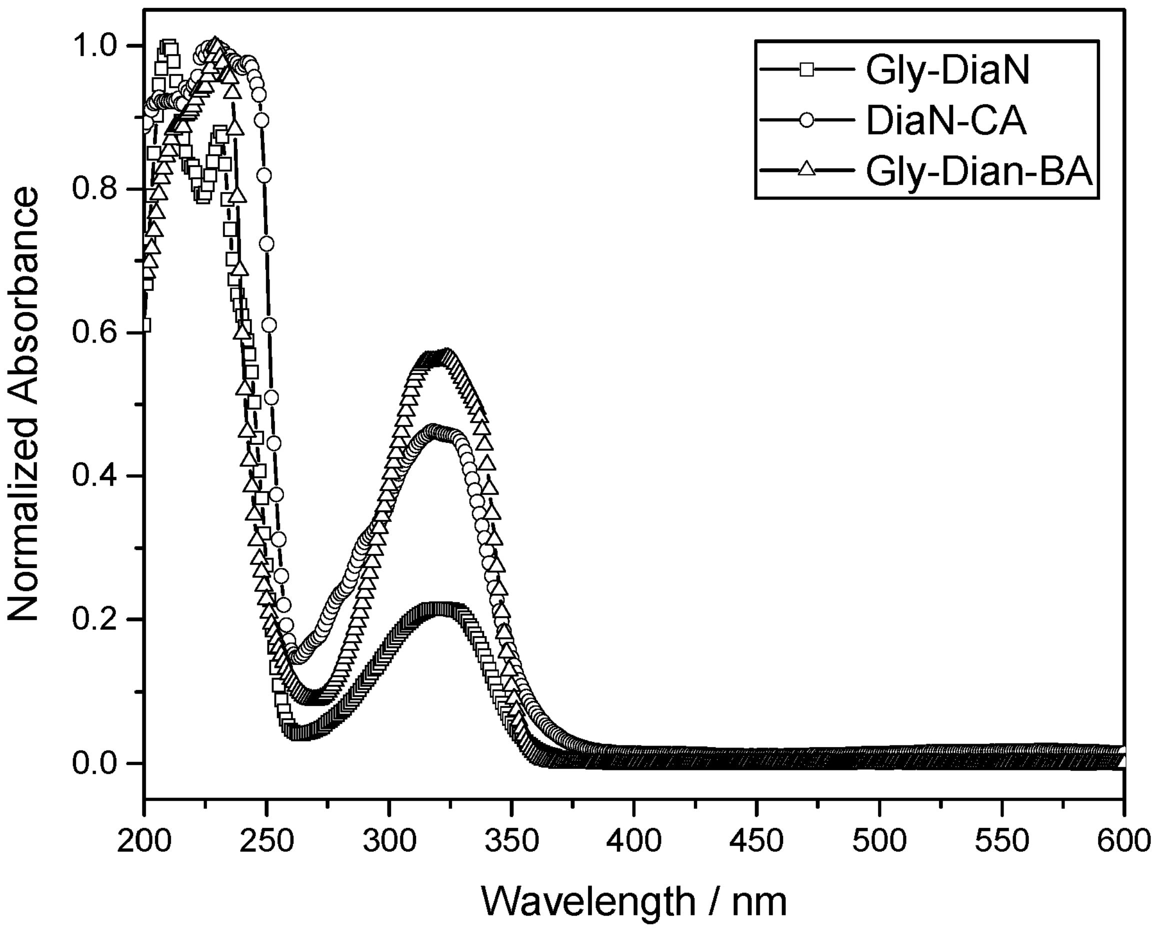
Gly-DiaN
DiaN-CA
Gly-Dian-BA
Normalized Absorbance
Wavelength / nm
1.0
0.8
0.6
0.4
0.2
0.0
200
250
300
350
400
450
500
550
600

Fig. 10

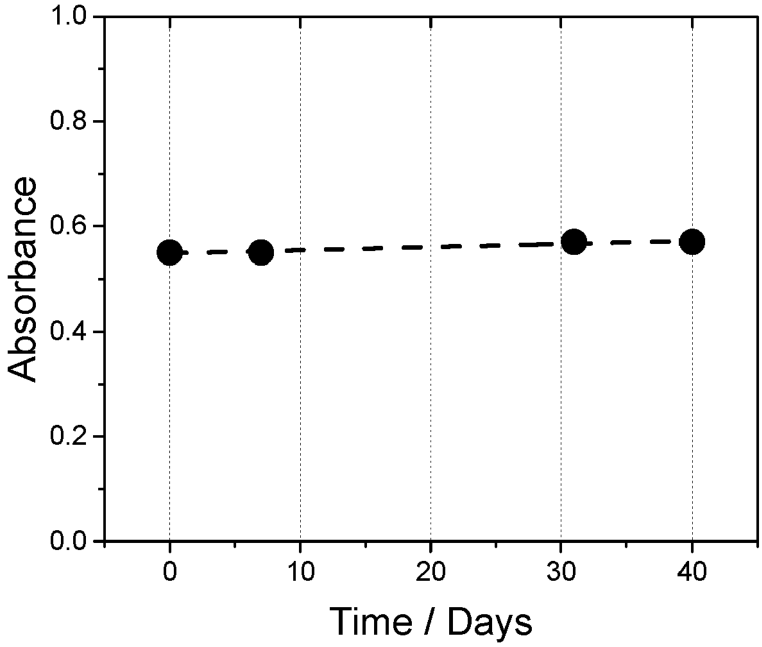
Absorbance
Time / Days
1.0
0.8
0.6
0.4
0.2
0.0
0
10
20
30
40

Fig. 11

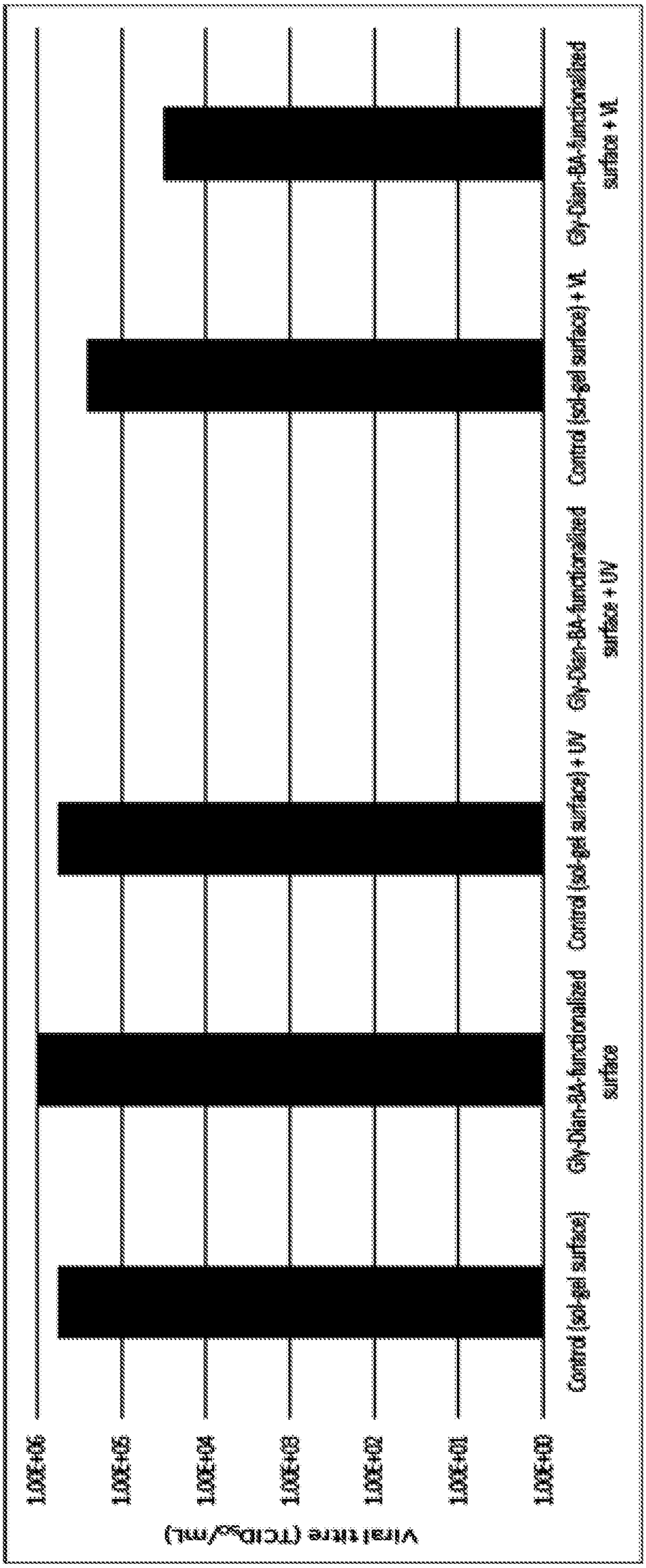
Viral titre (TCID50/mL)
1.00E+06
1.00E+05
1.00E+04
1.00E+03
1.00E+02
1.00E+01
1.00E+00
Control (sol-gel surface)
Gly-Dien-BA-functionalized surface
Control (sol-gel surface) + UV
Gly-Dien-BA-functionalized surface + UV
Control (sol-gel surface) + VL
Gly-Dien-BA-functionalized surface + VL

Fig. 12

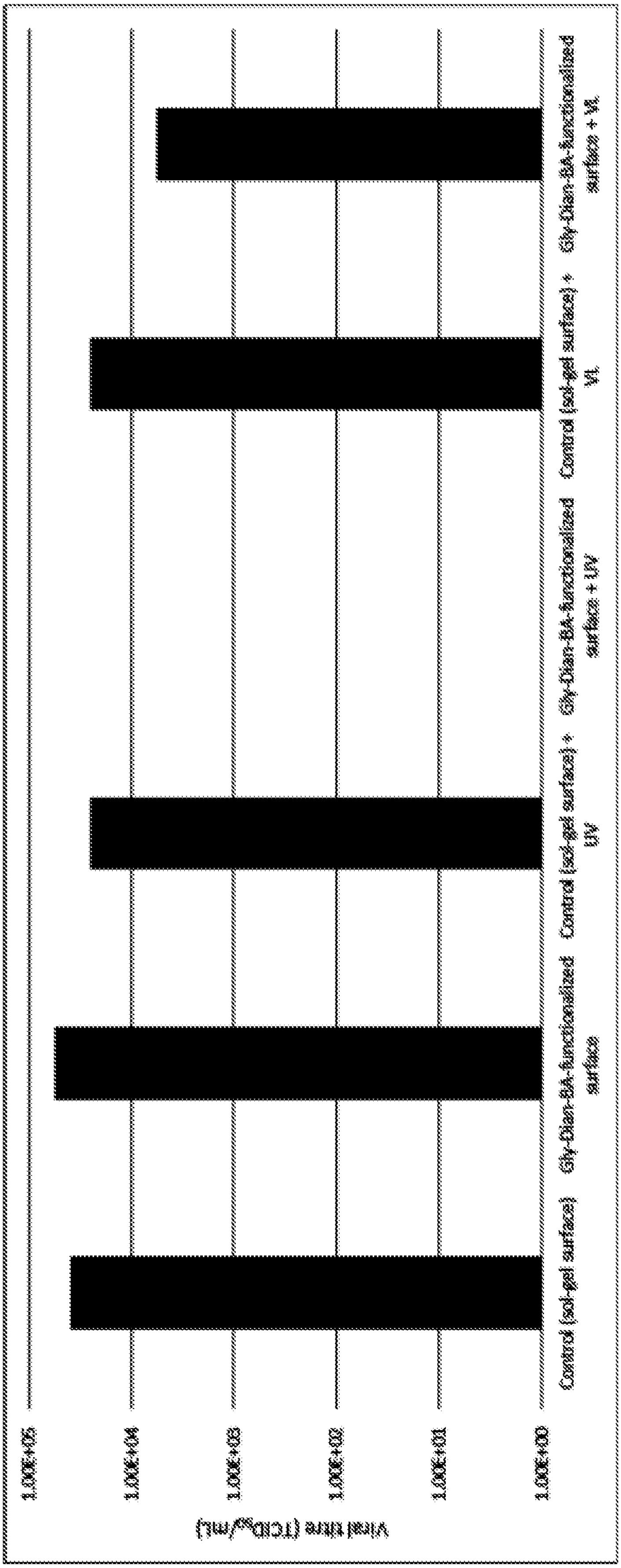
Viral titre (TCID50/mL)
1.00E+05
1.00E+04
1.00E+03
1.00E+02
1.00E+01
1.00E+00
Control (sol-gel surface)
Gly-Dian-BA-functionalized surface
Control (sol-gel surface) + UV
Gly-Dian-BA-functionalized surface + UV
Control (sol-gel surface) + VL
Gly-Dian-BA-functionalized surface + VL

Fig. 13

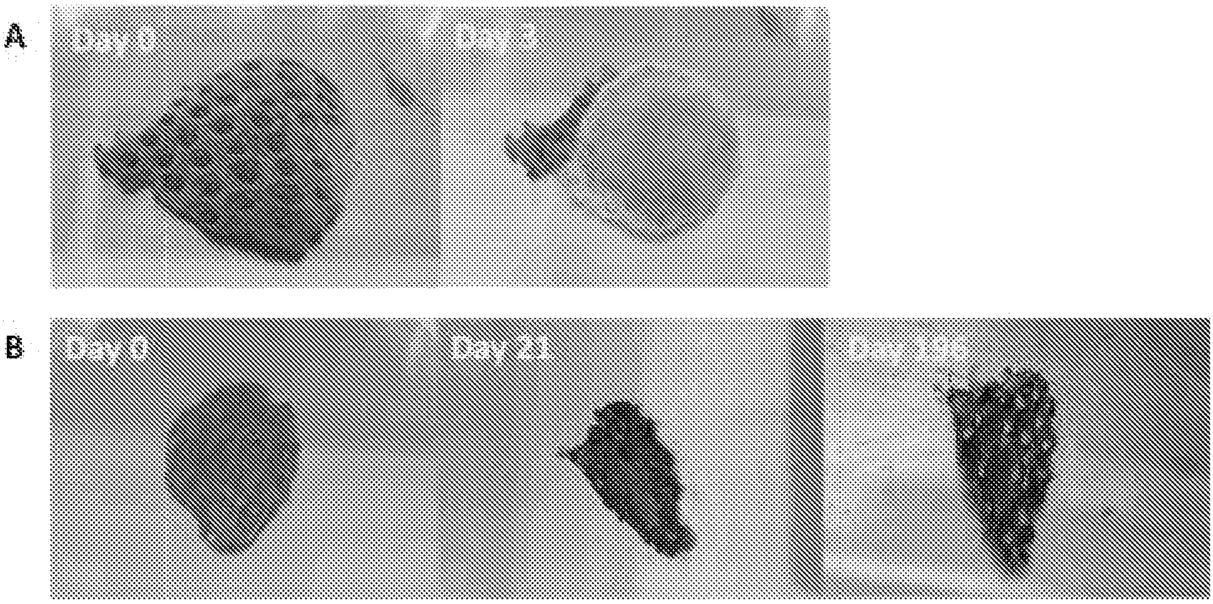


Fig. 14

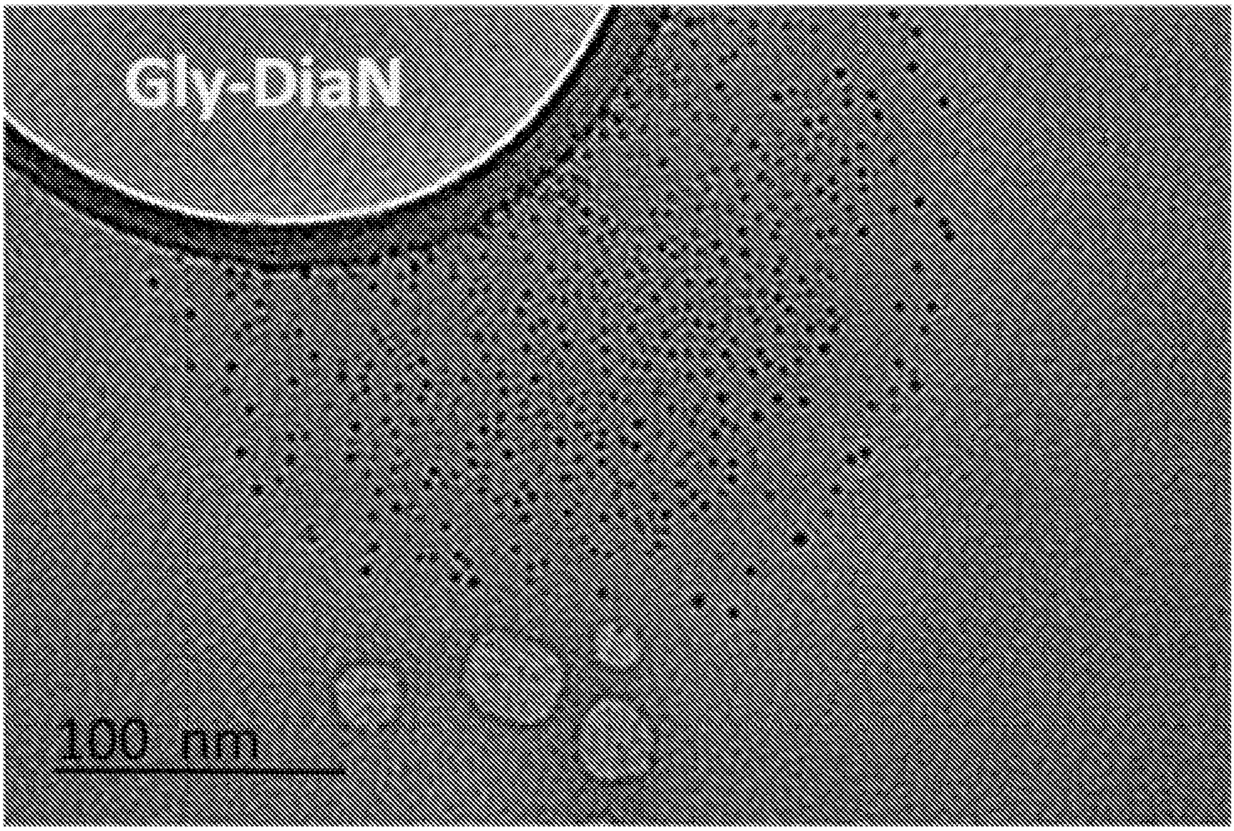


Fig. 15a

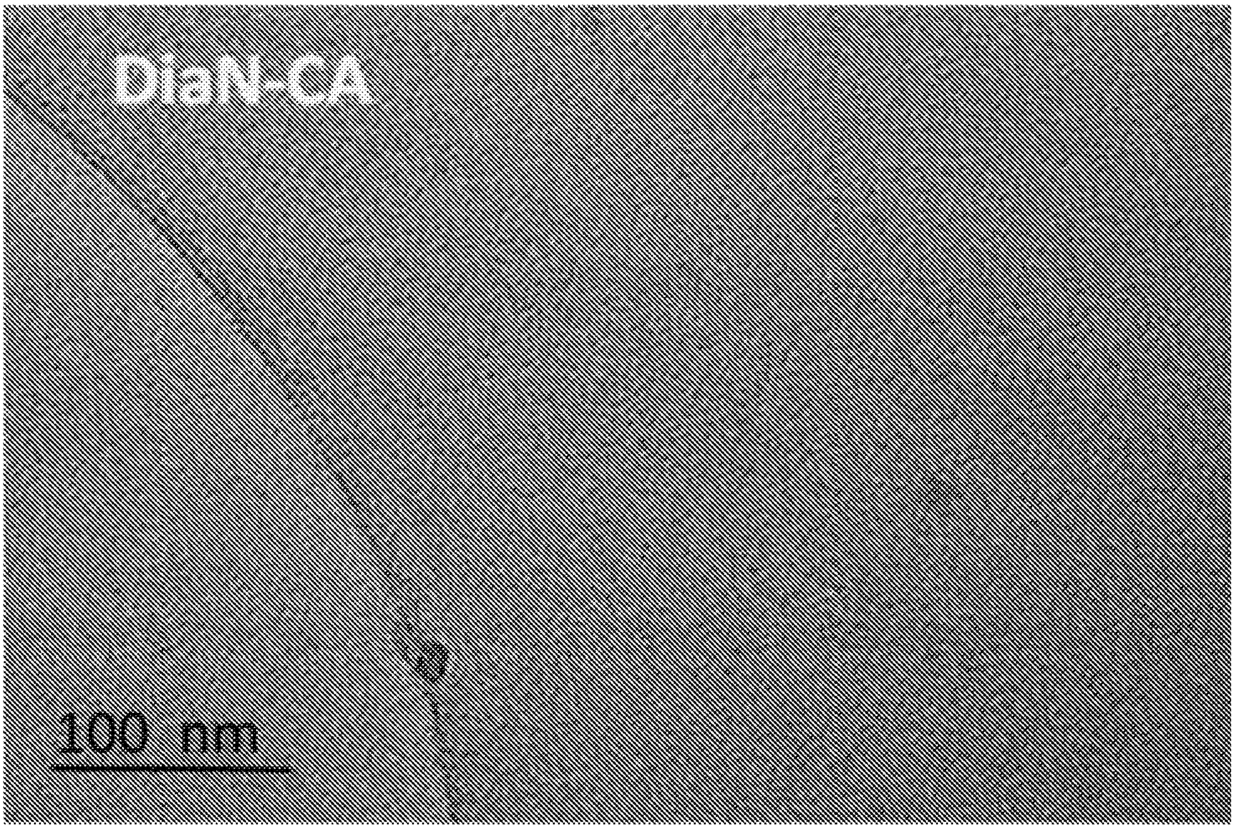


Fig. 15b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *ACS Biomater. Sci. Eng.*, 2018, vol. 4 (12), 3983-3993
- **MULLER-BREITKREUTZ**. *J Photochem PhotoBiol B*, 1995
- *Ogilby Chem Soc Rev*, 2010
- **B.Z. RISTIC et al.** *Biomaterials*, 2014, vol. 35, 4428-4435
- *ACS Nano*, 2016, vol. 10 (9), 8690-8699
- *Chem. Commun.*, 2017, vol. 53, 10588-10591
- **SHANAZ et al.** Oxidative Synthesis of Highly Fluorescent Boron/Nitrogen Co-Doped Carbon Nanodots Enabling Detection of Photosensitizer and Carcinogenic Dye. *Analytical Chemistry*, 2013, vol. 85 (21)
- **DAS et al.** Carbon Dots: An Emerging Smart Material for Analytical Application. *Micromachines*, 2021, vol. 2 (1), 84
- **KOVÁČOVÁ et al.** Carbon Quantum Dots Modified Polyurethane Nanocomposite as Effective Photocatalytic and Antibacterial Agents. *ACS Biomater. Sci. Eng.*, 2018, vol. 4 (12), 3983-3993
- **XIAOLIN NIE et al.** Carbon quantum dots: A bright future as photosensitizers for in vitro antibacterial photodynamic inactivation. *Photochem Photobiol B.*, 23 March 2020, vol. 206, 111864
- **INNOCENZI** ; **STAGI**. Carbon-based antiviral nanomaterials: graphene, C-dots, and fullerenes. A perspective. *Chem Sci.*, 14 July 2020, vol. 11 (26), 6606-6622
- **REED, L.J.** ; **MUENCH, H.** A simple method of estimating fifty percent endpoints. *The American Journal of Hygiene.*, 1938, vol. 27, 493-49